# EUROPEAN PATENT SPECIFICATION

(11) **EP 4 527 309 B1**
(45) Date of publication and mention of the grant of the patent: **08.04.2026**
(21) Application number: 24158712.0
(22) Date of filing: 20.02.2024
(51) Int. Cl.: A61B 8/00, G01S 7/52, A61B 8/08

(54) **WIRELESS ULTRASONIC PROBE**
DRAHTLOSE ULTRASCHALLSONDE
SONDE ULTRASONORE SANS FIL

(30) Priority: 25.09.2023 KR 20230128427
(43) Date of publication of application: 26.03.2025
(73) Proprietor: Samsung Medison Co., Ltd., Hongcheon-gun, Gangwon-do 25108 (KR)
(72) Inventor: YU, Hwanseung, 05790 Seoul (KR); LEE, Sangmok, 05597 Seoul (KR)
(74) Representative: Frey, Sven Holger

(56) References cited:
- WO-A1-2022/201655
- KR-B1- 101 940 917
- US-A1- 2019 110 777
- US-A1- 2023 371 926

## Description

### Technical Field

The disclosure relates to a wireless ultrasonic probe and an ultrasonic imaging system for obtaining ultrasonic images.

### Background Art

Recently, in a medical field, various medical imaging devices have been widely used to image and obtain information about biological tissues of a human body for the purpose of early diagnosis of various diseases or surgery. Representative examples of such medical imaging devices may include ultrasonic diagnostic devices, CT devices, and MRI devices.

An ultrasonic imaging device is a device that emits an ultrasonic signal generated from a transducer of a probe to an object, and non-invasively obtains at least one image of a region inside the object (e.g., soft tissue or blood flow) by receiving information from the signal reflected from the object. In particular, an ultrasonic diagnostic device is used for medical purposes such as observing the inside of an object, detecting foreign substances, and measuring injury. Such an ultrasonic diagnostic device is widely used along with other imaging diagnostic devices because the ultrasonic imaging device has higher stability than a diagnostic device using an X-ray, may display images in real time, and is safe because there is no radiation exposure.

In general, an ultrasonic imaging device may include a main body and an ultrasonic probe for transmitting ultrasonic signals to an object to be diagnosed and receiving signals reflected from the object.

Recently, as ultrasonic probes operating wirelessly have been commercialized, convenience is increasing.

However, because a wireless ultrasonic probe operates by being provided with limited electric power from a battery, the wireless ultrasonic probe has the disadvantage that its usage time is significantly reduced in operation modes that require high electric power, such as shear wave elastography (SWE) and continuous-wave doppler (CW) modes, which consume a lot of electric power.

In addition, because an internal space of a wireless ultrasonic probe is small, it is difficult for the wireless ultrasonic probe to implement a separate power source and a separate reception circuit required for operation modes such as the CW mode.
WO 2022/201655 A1 and US 2023/371926 A1 relate to an ultrasonic diagnostic apparatus and a control method for the ultrasonic diagnostic apparatus, and more particularly, to an ultrasonic diagnostic apparatus and an ultrasonic diagnostic apparatus in which an ultrasonic probe and an apparatus main body are used by switching between wired and wireless connection modes. The document also relates to a control method for a sound wave diagnostic apparatus.
US 2019/110777 A1 provides an ultrasound diagnosis apparatuses and methods of operating the same.

### Disclosure

### Technical Problem

The disclosure provides a wireless ultrasonic probe and an ultrasonic imaging system capable of supplying electric power depending on operation modes through an auxiliary power supply device capable of being coupled to the wireless ultrasonic probe, and providing a separate reception circuit.

### Technical Solution

The invention is defined by claim 1. In accordance with an aspect of the disclosure, a wireless probe includes a transmission module provided to transmit an ultrasonic signal to an object, a reception module provided to receive the ultrasonic signal reflected from the object, a main power source unit provided to supply electric power to the transmission module, an auxiliary power supply device including an auxiliary power source unit provided to supply electric power to the transmission module, and at least one processor configured to control the main power source unit or the auxiliary power source unit to supply electric power to the transmission module from the main power source unit or the auxiliary power source unit in response to a received a command to initiate an operating mode.

The at least one processor controls the main power source unit and the auxiliary power source unit to supply electric power to the transmission module from the auxiliary power source unit based on receiving a high electric power mode start command.

The high electric power mode may include a continuous-wave Doppler mode (hereinafter referred to as CW mode) or a shear wave elastography mode (hereinafter referred to as SWE mode).

The auxiliary power supply device is configured to be connected to or disconnected from the wireless ultrasonic probe by a user.

The main power source unit includes a main battery and a first switch provided to control the connection between the main battery and the transmission module, and the auxiliary power source unit includes an auxiliary battery, an output voltage variable module provided to vary a voltage of the electric power outputted from the auxiliary battery, a capacitor provided to charge electric energy for supplying electric power to the transmission module, a constant current circuit connected to the output voltage variable module to supply a constant current to the capacitor, a second switch provided to control the connection between the constant current circuit and the capacitor, and a third switch provided to control the connection between the capacitor and the transmission module.

A capacity of the auxiliary battery may be equal to or greater than a capacity of the main battery.

The at least one processor determines whether the connection of the auxiliary power supply device is activated based on receiving the high electric power mode start command,
determines whether predetermined charging conditions are satisfied to start charging the capacitor based on the connection of the auxiliary power supply device being activated, and turns on the first and second switches and turns off the third switch based on the charging conditions being satisfied.

The at least one processor may determine whether predetermined high electric power supply conditions are satisfied to use the electric energy charged in the capacitor after turning on the first and second switches and turning off the third switch, and turn off the first and second switches and turn on the third switch based on the high electric power supply conditions being satisfied.

The auxiliary power source unit may further include a discharge circuit provided to discharge the electric power charged in the capacitor and a fourth switch provided to control the connection between the discharge circuit and the capacitor.

The at least one processor may determine whether predetermined discharging conditions are satisfied to discharge the electric power charged in the capacitor after turning off the first and second switches and turning on the third switch, and turn off the first to third switches and turn on the fourth switch based on the discharging conditions being satisfied.

The wireless probe may further include a reception module including an amplifier provided to amplify the received echo signal, a main reception circuit provided to convert the echo signal amplified by the amplifier into a digital signal, and a fifth switch provided to control the connection between the amplifier and the main reception circuit, wherein the auxiliary power supply device may further include an auxiliary reception circuit provided to convert an echo signal into a digital signal in predetermined operation modes including the CW mode,
and a sixth switch provided to control the connection between the amplifier and the auxiliary reception circuit.

The at least one processor may determine whether the connection of the auxiliary power supply device is activated based on receiving a predetermined operation mode start command, and turn off the fifth switch and turn on the sixth switch based on the connection of the auxiliary power supply device being activated.

The at least one processor may determine whether an operation mode start command other than the predetermined operation mode is received based on receiving a predetermined operation mode termination command, and turn on the fifth switch and turn off the sixth switch based on receiving the operation mode start command other than the predetermined operation mode.

The at least one processor may turn off the fifth and sixth switches based on not receiving an operation mode start command other than the predetermined operation mode.

The at least one processor may transmit a control command to display the capacity of the auxiliary battery based on the connection of the auxiliary power supply device being activated.

The at least one processor may transmit a control command to deactivate an input interface related to the high electric power mode based on the connection of the auxiliary power supply device being deactivated.

In accordance with another aspect of the disclosure, an ultrasonic imaging system includes a wireless probe including a transmission module provided to transmit an ultrasonic signal to an object and a main battery provided to supply electric power to the transmission module, an auxiliary power supply device including an auxiliary battery provided to supply electric power to the transmission module and capable of being combined with the wireless probe, and at least one processor configured to control the main battery or the auxiliary battery to supply electric power to the transmission module from the main battery or the auxiliary battery in response to a received operation mode start command, wherein the at least one processor controls the wireless probe and the auxiliary power supply device to supply electric power to the transmission module from the auxiliary battery based on receiving a high electric power mode start command.

The high electric power mode may include a CW mode or a SWE mode.

The ultrasonic imaging system may further include at least one input interface, and at least one display, and the at least one processor may control the at least one display to display a capacity of the auxiliary battery based on the connection of the auxiliary power supply device being activated.

The at least one processor may control the at least one input interface to deactivate the at least one input interface related to the high electric power mode based on the connection of the auxiliary power supply device being deactivated.

### Advantageous Effects

According to an aspect of the disclosure, an operator can diagnose an object while having a wide range of motion using a wireless ultrasonic probe.

In addition, according to an aspect of the disclosure, the wireless ultrasonic probe can be provided with high electric power in operation modes such as a CW mode and a SWE mode through a combinable auxiliary power supply device.

In addition, according to an aspect of the disclosure, the wireless ultrasonic probe can be provided with a separate reception circuit required in a predetermined operation mode such as the CW mode through the combinable auxiliary power supply device.

### Description of Drawings

FIG. 1 illustrates a control block diagram of an ultrasonic imaging system 100 in a case in which a probe 20 is a wired probe or a hybrid probe;
FIG. 2 illustrates a control block diagram of the ultrasonic imaging system 100 in a case in which the probe 20 is a wired wireless probe or a hybrid probe;
FIGS. 3 to 6 are views illustrating the ultrasonic imaging system 100 according to an embodiment;
FIG. 7 is a view illustrating a form of combining the wireless probe 20 and an auxiliary power supply device 30 according to an embodiment;
FIG. 8 is a diagram illustrating a control block diagram of the auxiliary power supply device 30 according to an embodiment;
FIG. 9 is a block diagram for explaining the supply of electric power to a transmission module 113;
FIG. 10 is a block diagram for explaining a process of controlling at least one switch to charge electrical energy to a capacitor 326 in order to supply high electric power from an auxiliary battery 322 to the transmission module 113 according to an embodiment;
FIG. 11 is a block diagram for explaining a process of controlling at least one switch to supply high electric power to the transmission module 113 using electric energy charged in the capacitor 326 according to an embodiment;
FIG. 12 is a block diagram for explaining a process of controlling a plurality of switches in order to discharge electric energy charged in the capacitor 326 according to an embodiment;
FIG. 13 is a block diagram for explaining the connection of an amplifier 117a to one of a main reception circuit 117c and an auxiliary reception circuit 330 according to an embodiment;
FIG. 14 is a view illustrating a screen displayed on a display 140 according to an embodiment;
FIG. 15 is a view illustrating a screen displayed on the display 140 when the connection of the auxiliary power supply device 30 is activated according to an embodiment;
FIG. 16 is a view illustrating a screen displayed on the display 140 when the connection of the auxiliary power supply device 30 is deactivated according to an embodiment;
FIG. 17 is an overall control flowchart of the wireless probe 20 and the auxiliary electric power device 30 to supply electric power to the transmission module 113 according to an embodiment;
FIG. 18 is a control flowchart for controlling at least one switch to supply electric power to the transmission module 113 according to an embodiment; and
FIG. 19 is a control flowchart for controlling at least one switch to connect a transducer 115 to one of the main reception circuit 117c and the auxiliary reception circuit 330 according to an embodiment.

### Modes of the Invention

This disclosure will explain the principles and disclose embodiments of the disclosure to clarify the scope of the claims of the disclosure and enable those skilled in the art to which the embodiments of the disclosure belong to practice the embodiments. The embodiments of the disclosure may be implemented in various forms.

Like reference numbers refer to like elements throughout this specification. This specification does not describe all components of the embodiments, and general contents in the technical field to which the disclosure belongs or overlapping contents between the embodiments will not be described. The "module" or "unit" used in the specification may be implemented as one or a combination of two or more of software, hardware, or firmware, and according to embodiments, a plurality of "module" or "unit" may be implemented as a single element, or a single "module" or "unit" may include a plurality of elements.

The singular form of a noun corresponding to an item may include a single item or a plurality of items, unless the relevant context clearly indicates otherwise.

In this disclosure, each of phrases such as "A or B," "at least one of A and B," "at least one of A or B," "A, B or C," "at least one of A, B and C," and "at least one of A, B, or C" may include any one of the items listed together in the corresponding one of the phrases, or all possible combinations thereof.

The term "and/or" includes any combination of a plurality of related components or any one of a plurality of related components.

The terms such as "first," "second," "primary," and "secondary" may simply be used to distinguish a given component from other corresponding components, and do not limit the corresponding components in any other respect (e.g., importance or order).

The terms "front surface," "rear surface," "upper surface," "lower surface," "side surface," "left side," "right side," "upper portion," "lower portion," and the like used in the disclosure are defined with reference to the drawings, and the shape and position of each component are not limited by these terms.

The terms "comprises," "has," and the like are intended to indicate that there are features, numbers, steps, operations, components, parts, or combinations thereof described in the disclosure, and do not exclude the presence or addition of one or more other features, numbers, steps, operations, components, parts, or combinations thereof.

When any component is referred to as being "connected," "coupled," "supported," or "in contact" with another component, this includes a case in which the components are indirectly connected, coupled, supported, or in contact with each other through a third component as well as directly connected, coupled, supported, or in contact with each other.

When any component is referred to as being located "on" or "over" another component, this includes not only a case in which any component is in contact with another component but also a case in which another component is present between the two components.

Hereinafter, an ultrasonic device according to various embodiments will be described in detail with reference to the accompanying drawings. When described with reference to the attached drawings, similar reference numbers may be assigned to identical or corresponding components and redundant description thereof may be omitted.

In this disclosure, images may include a medical image obtained by a medical imaging device, such as a magnetic resonance imaging (MRI) device, a computed tomography (CT) device, an ultrasonic imaging device, and an x-ray imaging device.

In this disclosure, an 'object', which is subject to photography, may include a person, animal, or part thereof. For example, the object may include a part of a human body (organ, etc.) or a phantom.

Throughout this disclosure, an 'ultrasonic image' refers to an image of an object that has been processed based on an ultrasonic signal transmitted to and reflected from the object.

Hereinafter, embodiments will be described in detail with reference to the drawings.

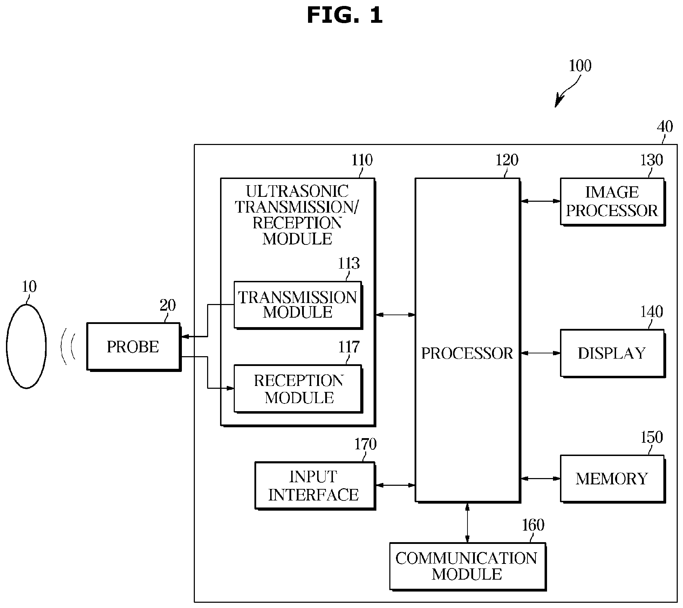

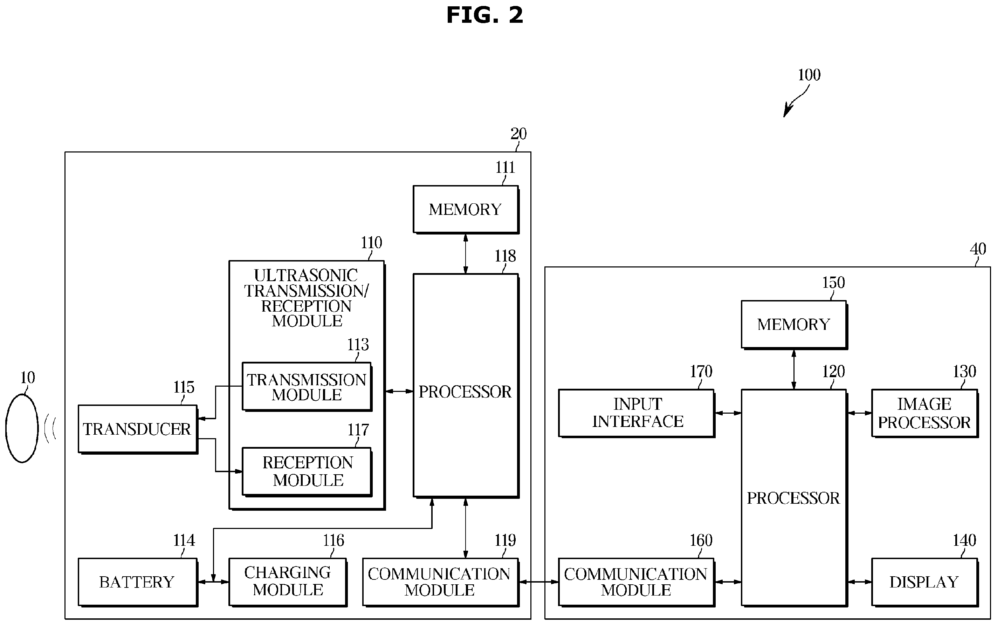

Referring to FIGS. 1 and 2, an ultrasonic imaging system 100 may include a probe 20 and an ultrasonic imaging device 40.

The ultrasonic imaging device 40 may be implemented not only in a cart type but also in a portable type. A portable ultrasonic imaging device may include, for example, a smart phone, laptop computer, PDA, tablet PC, etc., which include a probe and an application, but is not limited thereto.

The probe 20 may include a wired probe connected to the ultrasonic imaging device 40 by wire to communicate with the ultrasonic imaging device 40 by wire, a wireless probe wirelessly connected to the ultrasonic imaging device 40 to communicate wirelessly with the ultrasonic imaging device 40, and/or a hybrid probe by wire or wirelessly connected to the ultrasonic imaging device 40 to communicate by wire or wirelessly with the ultrasonic imaging device 40.

According to various embodiments, as illustrated in FIG. 1, the ultrasonic imaging device 40 may include an ultrasonic transmission/reception module 110, or as illustrated in FIG. 2, the probe 20 may include the ultrasonic transmission/reception module 110. According to various embodiments, both the ultrasonic imaging device 40 and the probe 20 may also include the ultrasonic transmission/reception module 110.

According to various embodiments, the probe 20 may further include an image processor 130, a display 140, and/or an input interface 170.

Accordingly, the descriptions of the ultrasonic transmission/reception module 110, the image processor 130, the display 140, and/or the input interface 170 included in the ultrasonic imaging device 40 may also be applied to the ultrasonic transmission/reception module 110, the image processor 130, the display 140, and/or the input interface 170 included in the probe 20.

FIG. 1 illustrates a control block diagram of the ultrasonic imaging system 100 in a case in which the probe 20 is a wired probe or a hybrid probe.

The probe 20 may include a plurality of transducers. The plurality of transducers may transmit an ultrasonic signal to an object 10 in response to a transmission signal applied from a transmission module 113. The plurality of transducers may form a received signal by receiving the ultrasonic signal (echo signal) reflected from the object 10. The probe 20 may be implemented as an integrated type with the ultrasonic imaging device 40, or may be implemented as a separate type connected to the ultrasonic imaging device 40 by wire. The ultrasonic imaging device 40 may be connected to the one or more probes 20 depending on the implementation type.

In the case in which the probe 20 is a wired probe or a hybrid probe, the probe 20 may include a cable and a connector capable of being connected to a connector of the ultrasonic imaging device 40.

The probe 20 according to an embodiment may be implemented as a two-dimensional probe. In a case in which the probe 20 is implemented as a two-dimensional probe, the plurality of transducers included in the probe 20 may be arranged in two dimensions to form a two-dimensional transducer array.

For example, the two-dimensional transducer array may have a form in which a plurality of sub-arrays including the plurality of transducers arranged in a first direction is arranged in a second direction different from the first direction.

In addition, in the case in which the probe 20 is implemented as a two-dimensional probe, the ultrasonic transmission/reception module 110 may include an analog beamformer and a digital beamformer. Alternatively, the two-dimensional probe may include one or both of the analog beamformer and the digital beamformer depending on the implementation type.

A processor 120 controls the transmission module 113 to form a transmission signal to be applied to each of the transducers 115 in consideration of positions and focused points of the plurality of transducers included in the probe 20.

The processor 120 may control a reception module 117 to generate ultrasonic data by converting reception signals received from the probe 20 to analog to digital and summing up the digitally converted reception signals in consideration of the positions and focused points of the plurality of transducers.

In the case in which the probe 20 is implemented as a two-dimensional probe, the processor 120 may calculate a time delay value for digital beamforming for each sub-array for each of the plurality of sub-arrays included in the two-dimensional transducer array. The processor 120 may also calculate a time delay value for analog beamforming for each of the transducers included in one of the plurality of sub-arrays. The processor 120 may control the analog beamformer and the digital beamformer to form a transmission signal to be applied to each of the plurality of transducers depending on the time delay values for analog beamforming and the time delay values for digital beamforming. The processor 120 may also control the analog beamformer to sum up the signals received from the plurality of transducers for each sub-array depending on the time delay values for analog beamforming. The processor 120 may also control the ultrasonic transmission/reception module 110 to convert the summed signal for each sub-array to analog to digital. The processor 120 may also control the digital beamformer to generate ultrasonic data by summing up the digitally converted signals depending on the time delay values for digital beamforming.

The image processor 130 generates an ultrasonic image using the generated ultrasonic data.

The display 140 may display the generated ultrasonic image and a variety of information processed by the ultrasonic imaging device 40 and/or the probe 20. The probe 20 and/or the ultrasonic imaging device 40 may include the one or more displays 140 depending on the implementation type. The display 140 may also include a touch panel or a touch screen.

The processor 120 may control the overall operation of the ultrasonic imaging device 40 and signal flow between internal components of the ultrasonic imaging device 40. The processor 120 may perform or control various operations or functions of the ultrasonic imaging device 40 by executing programs or instructions stored in a memory 150. The processor 120 may also control an operation of the ultrasonic imaging device 40 by receiving a control signal from the input interface 170 or an external device.

The ultrasonic imaging device 40 may include a communication module 160, and may be connected to an external device (e.g., the probe 20, a server, medical device, portable device (a smart phone, tablet PC, wearable device, etc.)) through the communication module 160.

The communication module 160 may include one or more components that enable communication with the external device, and may include, for example, at least one of a short-range communication module, a wired communication module, and a wireless communication module.

The communication module 160 may receive a control signal and data from the external device, and may transmit the received control signal to the processor 120 to enable the processor 120 to control the ultrasonic imaging device 40 depending on the received control signal.

Alternatively, the processor 120 may transmit a control signal to the external device through the communication module 160 to control the external device depending on the control signal of the processor.

For example, the external device may process data within the external device depending on the control signal of the processor received through the communication module.

A program capable of controlling the ultrasonic imaging device 40 may be installed in the external device, and this program may include instructions for performing some or all of the operations of the processor 120.

The program may be pre-installed on the external device, or a user of the external device may download and install the program from a server providing an application. The server providing the application may include a recording medium in which the program is stored.

The memory 150 may store various data or programs for driving and controlling the ultrasonic imaging device 40, inputted and outputted ultrasonic data, ultrasonic images, etc.

The input interface 170 may receive user input for controlling the ultrasonic imaging device 40. For example, the user input may include, but is not limited to, input of manipulating a button, a keypad, a mouse, a trackball, a jog switch, a knob, and the like, input of touching a touch pad or touch screen, voice input, motion input, biometric information input (e.g., iris recognition, fingerprint recognition, etc.), and the like.

FIG. 2 illustrates a control block diagram of the ultrasonic imaging system 100 in the case in which the probe 20 is a wireless probe or a hybrid probe.

According to various embodiments, the ultrasonic imaging device 40 illustrated in FIG. 2 may be replaced with the ultrasonic imaging device 40 described with reference to FIG. 1.

According to various embodiments, the probe 20 illustrated in FIG. 1 may be replaced with the probe 20 to be described with reference to FIG. 2.

The probe 20 may include the transmission module 113, a battery 114, the transducer 115, a charging module 116, the reception module 117, a processor 118, and a communication module 119. Although FIG. 2 illustrates that the probe 20 includes both the transmission module 113 and the reception module 117, the probe 20 may include only part of a configuration of the transmission module 113 and the reception module 117 depending on the implementation type, and the part of the configuration of the transmission module 113 and the reception module 117 may be included in the ultrasonic imaging device 40. Alternatively, the probe 20 may further include the image processor 130.

The transducer 115 may include a plurality of transducers. The plurality of transducers may transmit an ultrasonic signal to the object 10 in response to a transmission signal applied from the transmission module 113. The plurality of transducers may receive the ultrasonic signal reflected from the object 10 to form a reception signal.

The charging module 116 may charge the battery 114. The charging module 116 may receive electric power from the outside. The charging module 116 may receive electric power wirelessly. However, the disclosure is not limited thereto, and the charging module 116 may receive electric power by wire. The charging module 116 may transfer the received electric power to the battery 114.

The processor 118 controls the transmission module 113 to form a transmission signal to be applied to each of the plurality of transducers in consideration of the positions and focused points of the plurality of transducers.

The processor 118 controls the reception module 117 to generate ultrasonic data by converting reception signals received from the transducer 115 to analog to digital and summing up the digitally converted reception signals in consideration of the positions and focused points of the plurality of transducers. Alternatively, in a case in which the probe 20 includes the image processor 130, the probe 20 may generate an ultrasonic image using the generated ultrasonic data.

In the case in which the probe 20 is implemented as a two-dimensional probe, the processor 118 may calculate a time delay value for digital beamforming for each sub-array for each of the plurality of sub-arrays included in the two-dimensional transducer array. The processor 118 may also calculate a time delay value for analog beamforming for each of the transducers included in one of the plurality of sub-arrays. The processor 118 may control the analog beamformer and the digital beamformer to form a transmission signal to be applied to each of the plurality of transducers depending on the time delay values for analog beamforming and the time delay values for digital beamforming. The processor 118 may also control the analog beamformer to sum up the signals received from the plurality of transducers for each sub-array depending on the time delay values for analog beamforming. The processor 118 may also control the ultrasonic transmission/reception module 110 to convert the summed signal for each sub-array to analog to digital. The processor 118 may also control the digital beamformer to generate ultrasonic data by summing up the digitally converted signals depending on the time delay values for digital beamforming.

The processor 118 may control the overall operation of the probe 20 and the signal flow between the internal components of the probe 20. The processor 118 may perform or control the various operations or functions of the probe 20 by executing programs or instructions stored in a memory 111. The processor 118 may also control the operation of the probe 20 by receiving the control signal from the input interface 170 of the probe 20 or an external device (e.g., the ultrasonic imaging device 40).

The communication module 119 may wirelessly transmit the generated ultrasonic data or ultrasonic images to the ultrasonic imaging device 40 through a wireless network. The communication module 119 may also receive control signals and data from the ultrasonic imaging device 40.

The ultrasonic imaging device 40 may receive the ultrasonic data or ultrasonic images from the probe 20.

In an embodiment, in a case in which the probe 20 includes the image processor 130 capable of generating an ultrasonic image using the ultrasonic data, the probe 20 may transmit the ultrasonic data and/or the ultrasonic images generated by the image processor 130 to the ultrasonic imaging device 40.

In an embodiment, in a case in which the probe 20 does not include the image processor 130 capable of generating an ultrasonic image using the ultrasonic data, the probe 20 may transmit the ultrasonic data to the ultrasonic imaging device 40. The ultrasonic data may include ultrasonic raw data, and the ultrasonic image may refer to ultrasonic image data.

The ultrasonic imaging device 40 may include the processor 120, the image processor 130, the display 140, the memory 150, the communication module 160, and the input interface 170.

The image processor 130 generates an ultrasonic image using ultrasonic data received from the probe 20.

The display 140 may display an ultrasonic image received from the probe 20, an ultrasonic image generated by processing the ultrasonic data received from the probe 20, and a variety of information processed by the ultrasonic imaging system 100. The ultrasonic imaging device 40 may include the one or more displays 140 depending on the implementation type. The display 140 may include a touch panel or a touch screen.

The processor 120 may control the overall operation of the ultrasonic imaging device 40 and the signal flow between the internal components of the ultrasonic imaging device 40. The processor 120 may perform or control the various operations or functions of the ultrasonic imaging device 40 by executing the programs or applications stored in a memory 150. The processor 120 may also control the operation of the ultrasonic imaging device 40 by receiving the control signal from the input interface 170 or an external device.

The ultrasonic imaging device 40 may include the communication module 160, and may be connected to an external device (e.g., the probe 20, a server, medical device, portable device (a smart phone, tablet PC, wearable device, etc.)) through the communication module 160.

The communication module 160 may include one or more components that enable communication with the external device, and may include, for example, at least one of a short-range communication module, a wired communication module, and a wireless communication module.

The communication module 160 of the ultrasonic imaging device 40 and the communication module 119 of the probe 20 may communicate using a network or a short-range wireless communication method. For example, the communication module 160 of the ultrasonic imaging device 40 and the communication module 119 of the probe 20 may communicate using any one of wireless LAN, Wi-Fi, Bluetooth, ZigBee, Wi-Fi Direct (WFD), Infrared Data Association (IrDA), Bluetooth Low Energy (BLE), Near Field Communication (NFC), Wireless Broadband Internet (WiBro), World Interoperability for Microwave Access (WiMAX), Shared Wireless Access Protocol (SWAP), Wireless Gigabit Alliance (WiGig), RF communication, a wireless data communication method including 60GHz millimeter wave (mm wave) short-range communication, etc.

To this end, the communication module 160 of the ultrasonic imaging device 40 and the communication module 119 of the probe 20 may include at least one of a wireless LAN communication module, a Wi-Fi communication module, a Bluetooth communication module, a ZigBee communication module, a Wi-Fi Direct (WFD) communication module, an Infrared Data Association (IrDA) communication module, a Bluetooth Low Energy (BLE) communication module, a Near Field Communication (NFC) module, a Wireless Broadband Internet (WiBro) communication module, a World Interoperability for Microwave Access (WiMAX) communication module, a Shared Wireless Access Protocol (SWAP) communication module, a Wireless Gigabit Alliance (WiGig) communication module, a RF communication module, and a 60GHz millimeter wave (mm wave) short-range communication module.

In an embodiment, the probe 20 may transmit device information (e.g., ID information) of the probe 20 using a first communication method (e.g., BLE), may be wirelessly paired with the ultrasonic imaging device 40, and may transmit ultrasonic data and/or ultrasonic images to the paired ultrasonic imaging device 40.

The device information of the probe 20 may include a variety of information related to a serial number, model name, and battery state of the probe 20.

The ultrasonic imaging device 40 may receive the device information (e.g., ID information) of the probe 20 from the probe 20 using the first communication method (e.g., BLE), may be wirelessly paired with the probe 20, may transmit an activation signal to the paired probe 20, and may receive the ultrasonic data and/or ultrasonic images from the probe 20. In this case, the activation signal may include a signal for controlling the operation of the probe 20.

In an embodiment, the probe 20 may transmit the device information (e.g., ID information) of the probe 20 using the first communication method (e.g., BLE), may be wirelessly paired with the ultrasonic imaging device 40, and may transmit the ultrasonic data and/or ultrasonic images to the ultrasonic imaging device 40 paired by the first communication method using a second communication method (e.g., 60 GHz millimeter wave and Wi-Fi).

The ultrasonic imaging device 40 may receive the device information (e.g., ID information) of the probe 20 from the probe 20 using the first communication method (e.g., BLE), may be wirelessly paired with the probe 20, may transmit the activation signal to the paired probe 20, and may receive the ultrasonic data and/or ultrasonic images from the probe 20 using the second communication method (e.g., 60 GHz millimeter wave and Wi-Fi).

According to various embodiments, the first communication method used to pair the probe 20 and the ultrasonic imaging device 40 with each other may have a lower frequency band than a frequency band of the second communication method used by the probe 20 to transmit the ultrasonic data and/or ultrasonic images to the ultrasonic imaging device 40.

The display 140 of the ultrasonic imaging device 40 may display UIs indicating device information of the probe 20. For example, the display 140 may display UIs, which indicate identification information of the wireless probe 20, a pairing method indicating a pairing method with the probe 20, a data communication state between the probe 20 and the ultrasonic imaging device 40, a method of performing data communication with the ultrasonic imaging device 40, and the battery state of the probe 20.

In a case in which the probe 20 includes the display 140, the display 140 of the probe 20 may display UIs indicating the device information of the probe 20. For example, the display 140 may display UIs, which indicate the identification information of the wireless probe 20, the pairing method indicating the pairing method with the probe 20, the data communication state between the probe 20 and the ultrasonic imaging device 40, the method of performing data communication with the ultrasonic imaging device 40, and the battery state of the probe 20.

The communication module 160 may also receive a control signal and data from an external device and transmit the received control signal to the processor 120 so that the processor 120 controls the ultrasonic imaging device 40 depending on the received control signal.

Alternatively, the processor 120 may transmit a control signal to an external device through the communication module 160 to control the external device depending on the control signal of the processor 120.

For example, the external device may process data of the external device depending on the control signal of the processor 120 received through the communication module.

A program capable of controlling the ultrasonic imaging device 40 may be installed in the external device, and this program may include instructions for performing some or all of the operations of the processor 120.

The program may be pre-installed on the external device, or the user of the external device may download and install the program from the server providing the application. The server providing the application may include the recording medium in which the program is stored.

The memory 150 may store various data or programs for driving and controlling the ultrasonic imaging device 40, inputted and outputted ultrasonic data, ultrasonic images, etc.

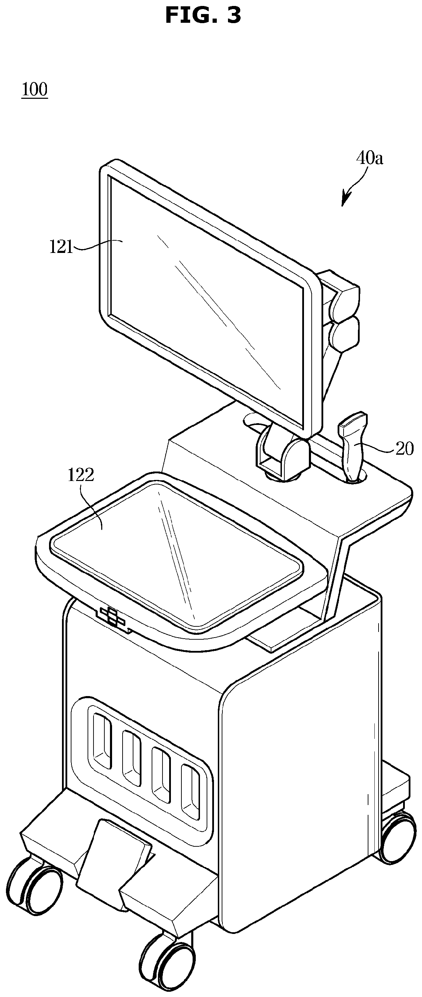

Examples of the ultrasonic imaging system 100 according to an embodiment of the disclosure will be described later through FIGS. 3, 4, 5, and 6.

FIGS. 3, 4, 5, and 6 are views illustrating ultrasonic imaging devices according to an embodiment.

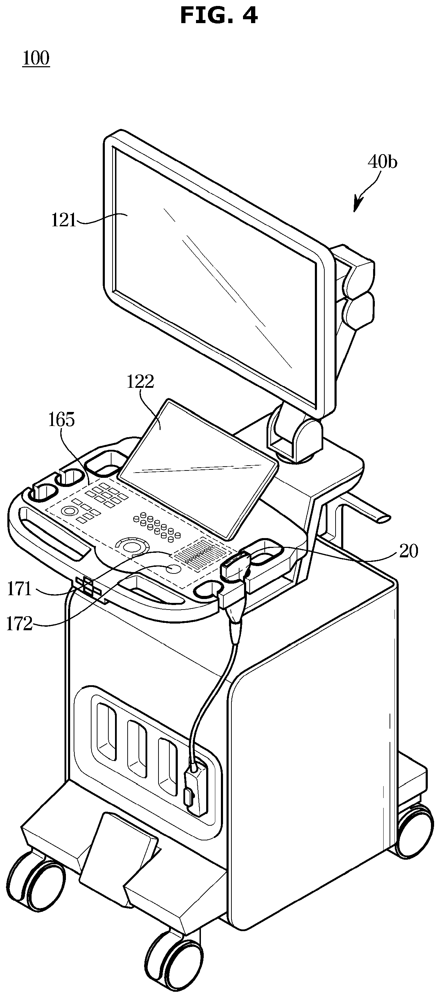

Referring to FIGS. 3 and 4, ultrasonic imaging devices 40a and 40b may include a main display 121 (140) and a sub display 122 (140). At least one of the main display 121 and the sub display 122 may be implemented as a touch screen. At least one of the main display 121 and the sub display 122 may display ultrasonic images or a variety of information processed by the ultrasonic imaging devices 40a and 40b. In addition, at least one of the main display 121 and the sub display 122 may be implemented as a touch screen and provide a GUI, so that data for controlling the ultrasonic imaging devices 40a and 40b may be inputted from the user. For example, the main display 121 may display an ultrasonic image, and the sub display 122 may display a control panel for controlling the display of the ultrasonic image in the form of a GUI. The sub display 122 may be provided with data for controlling the display of images through the control panel displayed in the form of a GUI. For example, a time gain compensation (TGC) button, a Freeze button, a trackball, a jog switch, a knob, and the like may be provided as a GUI on the sub display 122.

The ultrasonic imaging devices 40a and 40b may control the display of ultrasonic images displayed on the main display 121 using the inputted control data. The ultrasonic imaging devices 40a and 40b may also be connected to the probe 20 by wire or wirelessly to transmit and receive ultrasonic signals to and from the object 10.

Referring to FIG. 4, the ultrasonic imaging device 40b may further include a control panel 165 in addition to the main display 121 and the sub display 122. The control panel 165 may include a button, a trackball, a jog switch, a knob, and the like, and may be provided with data for controlling the ultrasonic imaging device 40b from the user. For example, the control panel 165 may include a time gain compensation (TGC) button 171, a Freeze button 172, and the like. The TGC button 171 is a button for setting a TGC value for each depth of the ultrasonic images. When the input of the Freeze button 172 is sensed while scanning an ultrasonic image, the ultrasonic imaging device 40b may maintain a state in which the frame image at that point in time is displayed.

A button, a trackball, a jog switch, a knob, and the like included in the control panel 165 may be provided as a GUI on the main display 121 or the sub display 122. The ultrasonic imaging devices 40a and 40b may be connected to the probe 20 to transmit and receive ultrasonic signals to and from the object 10.

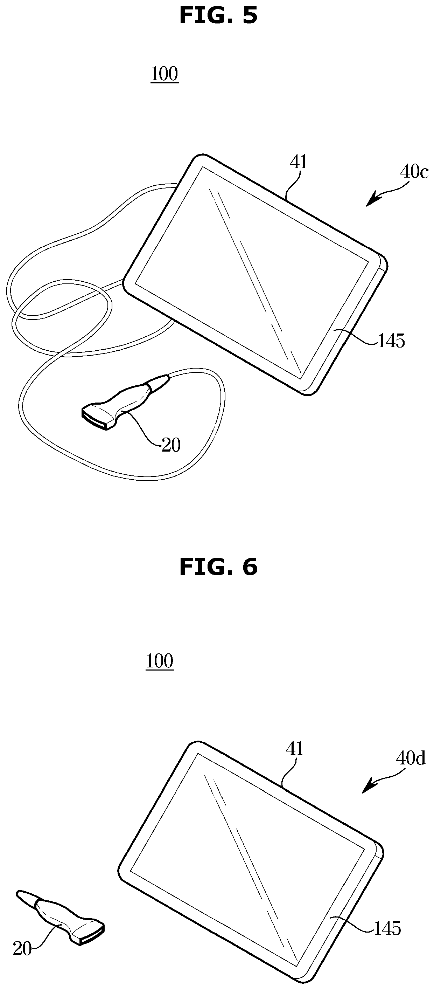

Referring to FIGS. 5 and 6, an ultrasonic imaging device 40c may also be implemented in a portable form. The portable ultrasonic imaging device 40c may include, for example, a smart phone, laptop computer, PDA, tablet PC, etc., which include a probe and an application, but is not limited thereto.

The ultrasonic imaging device 40c may include a main body 41. Referring to FIG. 5, the probe 20 may be connected to one side of the main body 41 by wire. To this end, the main body 41 may include a connection terminal through which a cable connected to the probe 20 may be attached and detached, and the probe 20 may include a connection terminal to and from which a cable connected to the main body 41 may be attached and detached.

Referring to FIG. 6, the probe 20 may be wirelessly connected to the ultrasonic imaging device 40c. The main body 41 may include an input/output interface (e.g., a touch screen) 145 (140 and 170). Ultrasonic images, a variety of information processed by the ultrasonic imaging devices, a GUI, and the like may be displayed on the input/output interface 145.

An ultrasonic image may be displayed on the input/output interface 145. The ultrasonic imaging device 40c may correct the ultrasonic image displayed on the input/output interface 145 using AI. The ultrasonic imaging device 40c may provide an alarm for informing using various audio-visual tools, such as graphics, sound, and vibration, information about lesions among the ultrasonic images displayed on the input/output interface 145 using AI.

The ultrasonic imaging device 40c may output a control panel displayed in the form of a GUI through the input/output interface 145.

An ultrasonic imaging device 40d and the probe 20 may perform communication or be paired using short-range wireless communication. For example, the ultrasonic imaging device 40d and the probe 20 may perform communication using Bluetooth, BLE, Wi-Fi, or Wi-Fi Direct.

The ultrasonic imaging devices 40c and 40d may execute a program or application related to the probe 20 to control the probe 20 and output information related to the probe 20. The ultrasonic imaging devices 40c and 40d may perform operations related to the probe 20 while communicating with a predetermined server. The probe 20 may be registered with the ultrasonic imaging devices 40c and 40d or may be registered with the predetermined server. The ultrasonic imaging devices 40c and 40d may communicate with the registered probe 20 and perform the operations related to the probe 20.

The ultrasonic imaging devices 40c and 40d may include various types of input/output interfaces such as speakers, LEDs, and vibration devices. For example, the ultrasonic imaging devices 40c and 40d may output a variety of information in the form of graphics, sound, or vibration through an input/output interface. The ultrasonic imaging devices 40c and 40d may also output various notifications or data through an input/output interface.

According to an embodiment of the disclosure, the ultrasonic imaging device 40a, 40b, 40c, or 40d may process ultrasonic images or obtain additional information from ultrasonic images, using an artificial intelligence (AI) model. According to an embodiment of the disclosure, the ultrasonic imaging device 40a, 40b, 40c, or 40d may generate an ultrasonic image or perform processing such as correction, image quality improvement, encoding, and decoding on the ultrasonic image, using the AI model. In addition, according to an embodiment of the disclosure, the ultrasonic imaging device 40a, 40b, 40c, or 40d may perform processing, such as baseline definition, anatomical information acquisition, lesion information acquisition, surface extraction, boundary definition, length measurement, area measurement, volume measurement, and annotation creation, from ultrasonic images using the AI model.

The AI model may be provided on the ultrasonic imaging device 40a, 40b, 40c, or 40d, or may be provided on the server.

The AI model may be implemented using various artificial neural network models or deep neural network models. In addition, the AI model may be learned and created using various machine learning algorithms or deep learning algorithms. The AI model may be implemented using, for example, a model such as a convolutional neural network (CNN), a recurrent neural network (RNN), a generative adversarial network (GAN), and a long short-term memory (LSTM).

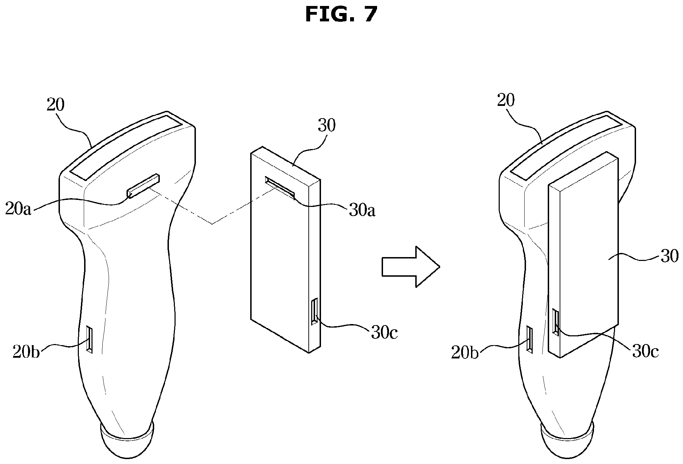

FIG. 7 is a view illustrating a form of combining the wireless probe 20 and an auxiliary power supply device 30 according to an embodiment.

Referring to FIG. 7, a first input terminal 20a and a second input terminal 20b for charging the main battery 114 (hereinafter, in order to distinguish between a battery of the wireless probe 20 and a battery 322 of the auxiliary power supply device 30, the battery of the wireless probe 20 will be referred to as the main battery 114 and the battery of the auxiliary power supply device 30 will be referred to as the auxiliary battery 322.) may be formed on one side surface of the wireless probe 20.

An output terminal 30a for supplying electric power to the transmission module 113 and a third input terminal 30c for charging the auxiliary battery 322 may be formed on one side surface of the auxiliary power supply device 30.

The positions where the first to third input terminals 20a, 20b, and 30c are formed are not limited to the disclosure, and the first to third input terminals 20a, 20b, and 30c may be adopted at any positions as long as the battery may be efficiently charged.

Also, the position where the output terminal 30a is formed is not limited to the disclosure, and the output terminal 30a may be adopted at any position as long as electric power may be efficiently supplied from the charged battery.

In this case, the first input terminal 20a and the output terminal 30a may be implemented in a form capable of being combined. As illustrated in FIG. 7, a connection protrusion may be formed and a connection groove capable of being fitted may be formed.

Any forms of the first input terminal 20a and the output terminal 30a may be adopted as long as the user may easily manipulate the first input terminal 20a and the output terminal 30a in a state of being combined.

As the first input terminal 20a of the wireless probe 20 is connected to the output terminal 30a of the auxiliary power supply device 30 through a connection line (not shown), the auxiliary power supply device 30 may supply electric power to the wireless probe 20.

The second input terminal 20b of the wireless probe 20 or the third input terminal 30c of the auxiliary power supply device 30 may charge the main battery 114 or the auxiliary battery 322, respectively, by being connected to a commercial electric power source through a connection line (not shown).

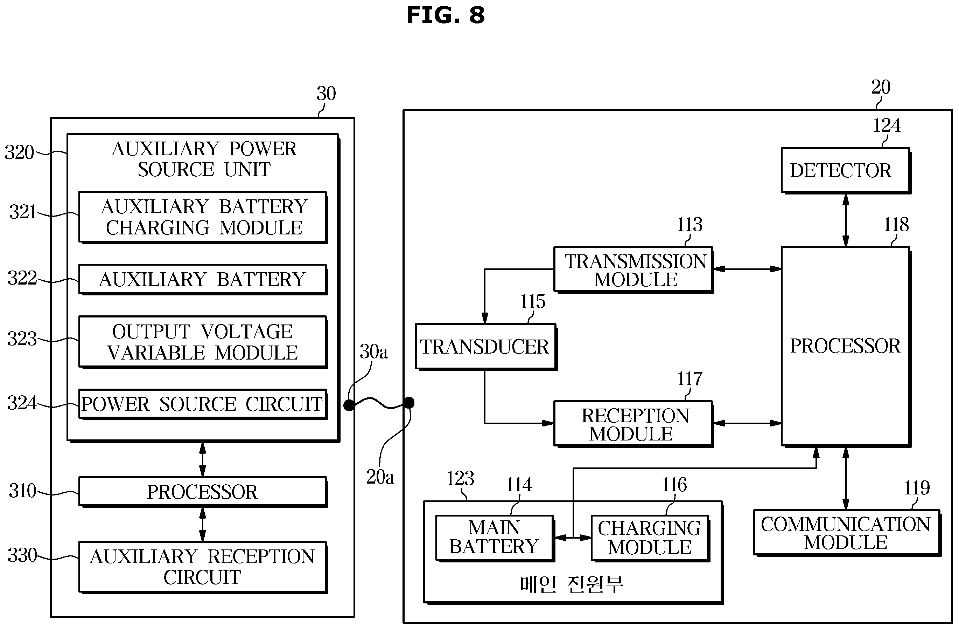

FIG. 8 is a diagram illustrating a control block diagram of the auxiliary power supply device 30 according to an embodiment.

According to various embodiments, the wireless probe 20 of FIG. 8 may also be replaced with the wireless probe 20 described with reference to FIG. 2.

In this case, the wireless probe 20 may include a main power source unit 123 including the main battery 114 and the charging module 116 capable of charging the main battery 114.

Referring to FIG. 8, the auxiliary power supply device 30 according to an embodiment may include an auxiliary power source unit 320 including an auxiliary battery charging module 321, the auxiliary battery 322, an output voltage variable module 323, and/or an power source circuit 324, and/or a processor 310 configured to control each component of the auxiliary power source unit 320.

The auxiliary battery charging module 321 may charge the auxiliary battery 322 by being provided with electric power from a commercial electric power source. One end of the auxiliary battery charging module 321 may be connected to the third input terminal 30c.

The auxiliary battery 322 may store electrical energy through charging and discharging and supply the stored electrical energy to an external device such as the wireless probe 20. The auxiliary battery 322 may include at least one battery cell, cables, and electrical components. The capacity of the auxiliary battery 322 may be larger than the capacity of the main battery 114 of the wireless probe 20.

The output voltage variable module 323 may be connected to the auxiliary battery 322 to adjust an output voltage of the auxiliary battery 322.

Because various electronic devices and systems require different operating voltages, the output voltage variable module 323 may enable the auxiliary power supply device 30 to interact with other external devices and efficiently supply electric power through output voltage conversion.

The output voltage variable module 323 may include a voltage regulator or switching regulator to adjust the output voltage.

The power source circuit 324 may be connected to the output voltage variable module 323, store electric energy in a capacitor 326 to supply high electric power, and supply the stored electric energy to the transmission module 113. A detailed description thereof will be provided later with reference to FIG. 8.

According to an embodiment, the auxiliary power supply device 30 may further include an auxiliary reception circuit 330.

The auxiliary reception circuit 330 may be a circuit that receives an echo signal amplified from the amplifier and converts the amplified echo signal into a digital signal instead of a main reception circuit 117c of the reception module 117 of the wireless probe 20 in a predetermined operation mode.

For example, the predetermined operation mode may be a CW mode.

In the case of the CW mode, the wireless probe 20 may determine a speed of a target 10 by transmitting an ultrasonic signal to the target 10 (e.g., a red blood cell), receiving the signal reflected from the target 10, and detecting a frequency shift of the received signal due to a movement of the target 10.

Therefore, in order for the wireless probe 20 to form a blood flow index based on speed data of the target, the separate auxiliary reception circuit 330 may be required to detect the frequency shift and derive the speed data.

When the wireless probe 20 is equipped with such a reception circuit, a problem arises in that the user may not manipulate the wireless probe 20 in detail as a size of the wireless probe 20 increases, but this problem may be solved by providing the auxiliary reception circuit 330 in the auxiliary power supply device 30 capable of being combined with the wireless probe 20.

The processor 310 may be electrically connected to the auxiliary power source unit 320 including the auxiliary battery charging module 321, the auxiliary battery 322, the output voltage variable module 323, and/or the power source circuit 324, and/or the auxiliary reception circuit 330.

That is, the processor 310 may control operations of the auxiliary power source unit 320.

For example, the processor 310 may control the output voltage variable unit 323 to adjust the output voltage of the auxiliary battery 322 based on an operation mode received by the wireless probe 20.

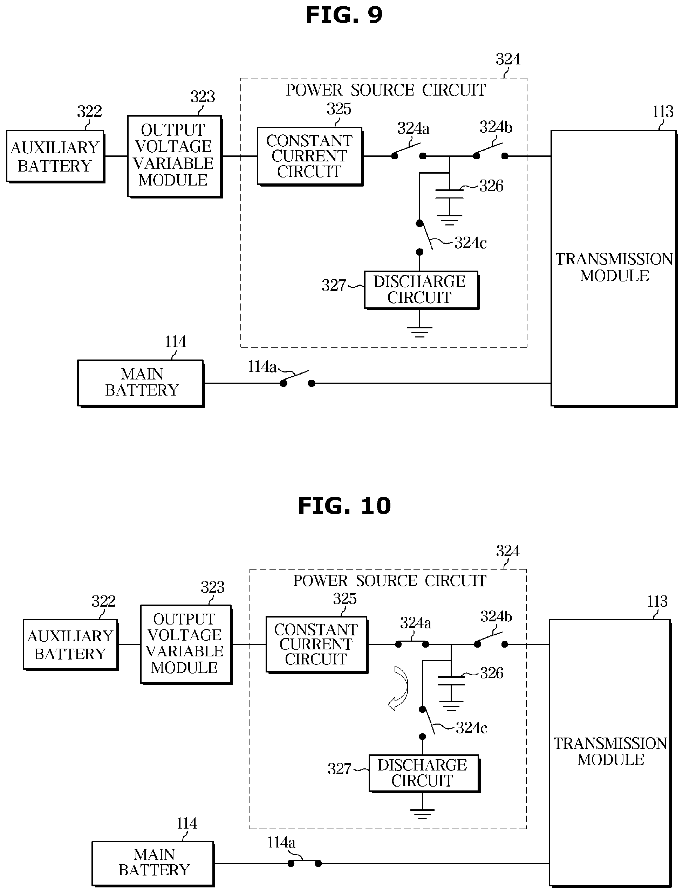

The processor 310 may also control a configuration of the power source circuit 324 to control a plurality of switches to supply electric power from the auxiliary battery 322 to the transmission module 113 through the power source circuit 324, and a detailed description thereof will be provided later with reference to FIGS. 9 to 12.

The processor 310 may also control the connection of the auxiliary reception circuit 330.

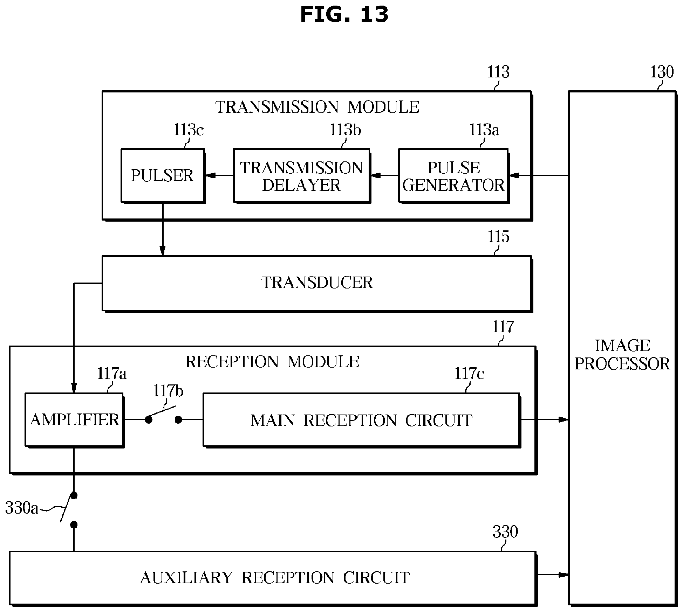

That is, the processor 310 may electrically connect or open the auxiliary reception circuit 330 and the amplifier based on the received information about the operation mode. A detailed description of controlling the connection of the auxiliary reception circuit 330 by controlling at least one switch of the auxiliary reception circuit 330 will be provided later with reference to FIG. 13.

Although it has been described above with reference to FIG. 8 that the one processor 310 controls the auxiliary power source unit 320 and/or the auxiliary reception circuit 330, a plurality of the processors may be provided.

In addition, the processor 118 of the wireless probe 20 may transmit a control command and the processor 310 of the auxiliary electric power device 30 may receive the control command, so that control of the auxiliary power source unit 320 and/or the auxiliary reception circuit 330 may be performed.

In addition, the processor 120 of the ultrasonic diagnostic device 40 may transmit a control command through the communication module 160, and the processor 310 of the wireless probe 20 that has received the control command may transmit the control command to the auxiliary electric power device 30.

Accordingly, at least one processor in the disclosure may include the processor 120 of ultrasonic diagnostic device 40, the processor 118 of wireless probe 20, and/or the processor 310 of auxiliary electric power device 30.

That is, control performed by the at least one processor may be refer to being performed by at least one of the processor 120 of the ultrasonic diagnostic device 40, the processor 118 of the wireless probe 20, and/or the processor 310 of the auxiliary electric power device 30.

In addition to the above-described configurations, the auxiliary power supply device 30 may include other configurations necessary for the auxiliary power supply device 30 to operate, or may be implemented with some of the above-described configurations omitted.

The power source circuit 324 of the auxiliary power supply device 30 to supply high electric power to the transmission module 113 based on the operation mode of the wireless probe 20 according to an embodiment will be described below with reference to FIGS. 9 to 12.

FIG. 9 is a block diagram for explaining the supply of electric power to the transmission module 113.

Referring to FIG. 9, the wireless probe 20 may include a first switch 114a configured to control the connection between the main battery 114 and the transmission module 113.

The at least one processor may control the first switch 114a to determine whether to supply electric power from the main battery 114 to the transmission module 113.

According to an embodiment, when high electric power is supplied from the auxiliary battery 322 to the transmission module 113, the first switch 114a may be turned off to block the supply of electric power from the main battery 113 to the transmission module 114.

Referring to FIG. 9, the power source circuit 324 may include a constant current circuit 325, a capacitor 326, a second switch 324a, and a third switch 324b. It will be understood by those skilled in the art that the power source circuit 320 may include other elements in addition to those illustrated in FIG. 9.

According to an embodiment, when the wireless probe 20 operates in a high electric power mode in which high electric power is required, the auxiliary power supply device 30 may supply high electric power to the transmission module 113.

In this case, in order to prevent distortion of a waveform of a pulse applied from the transmission module 113, which is supplied with electric power, to the transducer 115, a stable voltage needs to be supplied to the transmission module 113.

Therefore, in order for the transmission module 113 to operate properly and prevent overload of internal electronic components, electric power may be supplied using the constant current circuit 325.

The constant current circuit 325 is a circuit in which a constant current always flows regardless of a value of a voltage applied to both ends of the constant current circuit 325. Although a detailed configuration diagram of the constant current circuit 325 is not illustrates in FIG. 9, the constant current circuit 325 may be implemented with a certain transistor.

The constant current circuit 325 may be connected to the auxiliary battery 322 and supply electrical energy to the capacitor 326 to supply high electric power.

The capacitor 326 may charge electrical energy to supply high electric power. Herein, one side of the capacitor 326 may be connected to the auxiliary battery 322, and the other side of the capacitor 326 may be connected to the ground.

Although the disclosure illustrates that the one capacitor 326 is provided, the capacitor 326 may represent an equivalent capacitor for a plurality of the capacitors 326.

The second switch 324a may be positioned between the constant current circuit 325 and the capacitor 326 to control the connection of the constant current circuit 325 and the capacitor 326.

When the second switch 324a is turned on, the constant current circuit 325 and the capacitor 326 may be electrically connected, and when the second switch 324a is turned off, the constant current circuit 325 and the capacitor 326 may be electrically cut off.

That is, the second switch 324a may control the connection between the constant current circuit 325 and the capacitor 326 based on a control command of the at least one processor.

The third switch 324b may control the connection between the capacitor 326 and the transmission module 113.

When the third switch 324b is turned on, the capacitor 326 and the transmission module 113 may be electrically connected, and when the third switch 324b is turned off, the capacitor 326 and the transmission module 113 may be electrically cut off.

That is, the third switch 324b may control the connection between the capacitor 326 and the transmission module 113 based on the control command of the at least one processor.

The power source circuit 324 may turn on the second switch 324a to connect the constant current circuit 325 to the capacitor 326, and turn off the third switch 324b to cut off the connection between the capacitor 326 and the transmission circuit 113. In this case, a capacitor 326 may charge electrical energy based on a current supplied from a constant current circuit 325.

Also, the power source circuit 324 may turn off the second switch 324a to cut off the connection between the constant current circuit 325 and the capacitor 326, and turn on the third switch 324b to connect between the capacitor 326 and the transmission circuit 113. In this case, the electrical energy stored in the capacitor 326 may be supplied to the transmission module 113.

The power source circuit 324 may further include a discharge circuit 327 and a fourth switch 324c in addition to the constant current circuit 325, the capacitor 326, the second switch 324a, and the third switch 324b.

When the electric energy charged in the capacitor 326 in the power source circuit 324 is not discharged and continues to remain in the capacitor 326, the auxiliary power supply device 30 may be continuously loaded, and the lifespan of the capacitor 326 may be shortened.

The discharge circuit 327 may improve the durability of the auxiliary power supply device 30 by discharging the electric energy charged in the capacitor 326.

One end of the discharge circuit 327 may be connected to the ground. It will be understood by those skilled in the art that the discharge circuit 327 may include other elements in addition to those illustrated in FIG. 9.

The fourth switch 324c may be positioned between the discharge circuit 327 and the capacitor 326 to control the connection between the discharge circuit 327 and the capacitor 326.

The fourth switch 324c may be connected to a node between the second switch 324a and the third switch 324b.

The power source circuit 324 may turn on the fourth switch 324c to connect the capacitor 326 to the discharge circuit 327 and discharge the electric energy charged in the capacitor 326.

Referring to FIG. 9, the wireless probe 20 may include the first switch 114a to control the connection between the main battery 114 and the transmission module 113.

According to an embodiment, when high electric power is supplied from the auxiliary battery 322 to the transmission module 113, the first switch 114a is turned off to block the supply of electric power from the main battery 113 to the transmission module 114.

In this case, only the supply of electric power to the transmission module 113 is blocked, and the main battery 114 may supply electric power to components of the wireless probe 20 other than the transmission module 113.

FIG. 10 is a block diagram for explaining a process of controlling at least one switch to charge electrical energy to the capacitor 326 in order to supply high electric power from the auxiliary battery 322 to the transmission module 113 according to an embodiment.

Specifically, as the wireless probe 20 operates in the high electric power mode, the auxiliary battery 322 may supply electrical energy to the capacitor 326 through the constant current circuit 325 in the power source circuit 324.

The power source circuit 324 may charge the capacitor 326 with an amount of charge based on a current supplied from the constant current circuit 325 within the power source circuit 324. In this case, the capacitor 326 may be charged with an amount of charge in proportion to the capacity of the capacitor 326.

The at least one processor may determine whether the connection of the auxiliary power supply device 30 is activated based on receiving a command to initiated a high electric power mode, and determine whether predetermined charging conditions are satisfied in order to start charging the capacitor 326 when the connection of the auxiliary power supply device 30 is activated.

In this case, that the connection of the auxiliary power supply device 30 is activated may refer to a state in which the auxiliary power supply device 30 and the wireless probe 20 are combined and electric power may be supplied to the transmission module 113.

In this case, the predetermined charging conditions may include that the command to initiated the high electric power mode is received and that the connection of the auxiliary power supply device 30 is activated.

In addition, the predetermined charging conditions may include that the command to initiated the high electric power mode is received, that the connection of the auxiliary power supply device 30 is activated, and that a charging capacity of the main battery 114 has a value equal to or less than a predetermined value.

The at least one processor may turn on the second switch 324a and turn off the third switch 324b based on satisfying the above-described charging conditions.

For example, the at least one processor may determine whether a command for starting the high electric power mode is received through the input interface 170, and determine whether the auxiliary power supply device 30 is connected to the wireless probe 20 by a detector 124.

The at least one processor may receive the command to initiated the high electric power mode, and turn on the second switch 324a and turn off the third switch 324b when the auxiliary power supply device 30 is connected.

In addition, the at least one processor may receive the command to initiated the high electric power mode, and turn on the second switch 324a and turn off the third switch 324b when the charging capacity of the main battery 114 becomes lower than 50 percentages of the full charging capacity after the auxiliary power supply device 30 is connected.

In this case, the case in which the charging capacity of the main battery 114 becomes lower than 50 percentages of the full charging capacity is only an example, and a capacity of the main battery 114 from which control of the second switch 324a and the third switch 324b begins may be predetermined by the user as a predetermined value.

When the second switch 324a is turned on and the third switch 324b is turned off by the at least one processor, electrical energy may be charged to the capacitor 326 based on a constant current supplied from the constant current circuit 325.

In this case, in a case in which the capacitor 326 of the power source circuit 324 is charging, the at least one processor may turn on the first switch 114a so that electric power is supplied from the main battery 114 to the transmission module 113.

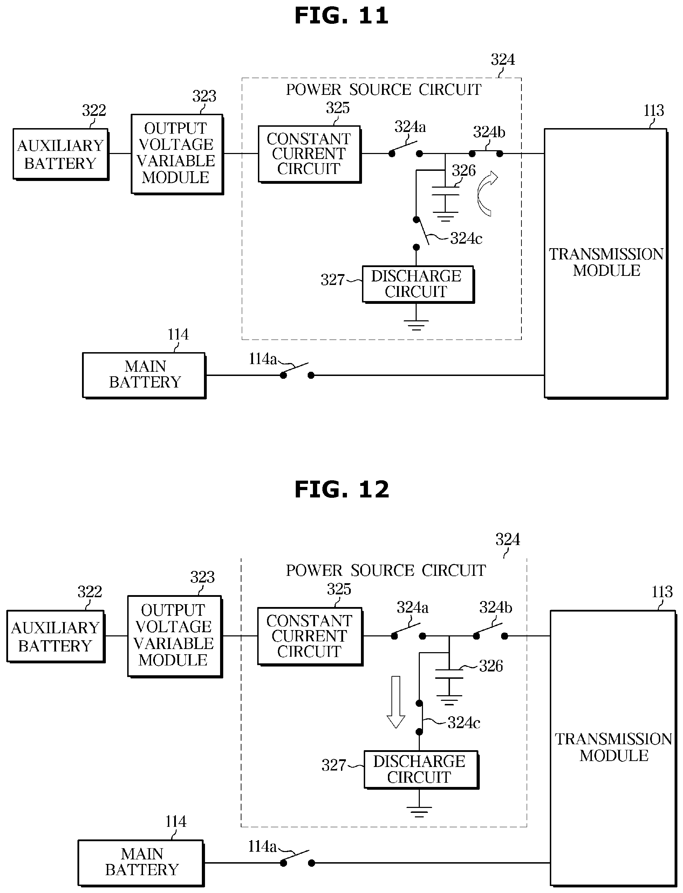

FIG. 11 is a block diagram for explaining a process of controlling at least one switch to supply high electric power to the transmission module 113 using electric energy charged in the capacitor 326 according to an embodiment.

Specifically, the at least one processor may control the power source circuit 324 to turn off the second switch 324a to block the connection between the constant current circuit 325 and the capacitor 326, and to turn on the third switch 324b to connect the capacitor 326 and the transmission module 113.

In addition, the at least one processor may turn off the first switch 114a to block electric power from being supplied from the main battery 114 to the transmission module 113.

That is, the at least one processor may control the power source circuit 324 such that high electric power is supplied to the transmission module 113 using the electric energy of the capacitor 326 in the power source circuit 324.

When the charging of electric energy to the capacitor 326 in the power source circuit 324 is completed, the power source circuit 324 may supply high electric power to the transmission module 113 based on the charged electric energy. That is, the at least one processor may control elements in the power source circuit 324 such that the operation of the power source circuit 324 is changed from an electric energy charging operation to a high electric power supply operation.

After turning on the second switch 324a and turning off the third switch 324b, the at least one processor may determine whether predetermined high electric power supply conditions are satisfied to use the electric energy charged in the capacitor 326.

In this case, the predetermined high electric power supply conditions may include that the wireless probe 20 starts operating or is operating in the high electric power mode and that the charging of the capacitor 326 is completed.

In addition, the predetermined high electric power supply conditions may include that a predetermined period of time has passed since the wireless probe 20 operates in the high electric power mode. For example, after ten minutes have elapsed from the time the command to initiated the high electric power mode is received, the at least one processor may turn off the first switch 114a to block supply of electric power from the main battery 114 to the transmission module 113, turn off the second switch 324a to block the connection between the constant current circuit 325 and the capacitor 326, and turn on the third switch 324b to connect the capacitor 326 and the transmission module 113.

That is, depending on operation control of the first switch 114a, the second switch 324a, and the third switch 324b, the at least one processor may supply high electric power to the transmission module 113 based on the electric energy charged in the capacitor 326 of the power source circuit 324.

FIG. 12 is a block diagram for explaining a process of controlling a plurality of switches in order to discharge electric energy charged in the capacitor 326 according to an embodiment.

When the wireless probe 20 operates in a mode other than the high electric power mode or enters an idle state in which it does not operate in any mode, the at least one processor may discharge the electrical energy charged in the capacitor 326 through the discharge circuit 327.

Referring to FIG. 12, when receiving a high electric power mode termination command, the at least one processor may control the discharge circuit 327 to turn on the fourth switch 324c to connect the capacitor 326 to the ground, thereby discharging the electric energy charged in the capacitor 326.

The at least one processor may turn off the second switch 324a and the third switch 324b in the power source circuit 324 so that electrical energy is not charged to the capacitor 326 and the electrical energy charged in the capacitor 326 is not supplied to the transmission module 113.

In this case, after the high electric power mode termination command is received, the at least one processor may control the first switch 114a by determining whether a start input related to a mode other than the high electric power mode is received.

When the start input related to a mode other than the high electric power mode is received after the high electric power mode is terminated, the at least one processor may turn on the first switch 114a so that electric power may be supplied to the transmission module 113.

On the other hand, when the high electric power mode is terminated and no mode start command is received, that is, in the idle state, the at least one processor may turn off the first switch 114a so that electric power is not supplied to the transmission module 113.

It has been described above with reference to FIGS. 9 to 12 that the at least one processor controls a plurality of switches to supply electric power to the transmission module 113.

Hereinafter, the auxiliary reception circuit 330 that the auxiliary power supply device 30 may additionally include will be described with reference to FIG. 13.

FIG. 13 is a block diagram for explaining the connection of an amplifier 117a to one of the main reception circuit 117c and the auxiliary reception circuit 330 according to an embodiment.

FIG. 13 is a block diagram illustrating the configurations of the transmission and reception modules 113 and 117, and the transducer 115 of the wireless probe 20, the transducer 115, and the auxiliary reception circuit 330 of the auxiliary power supply device 30.

The wireless probe 20 may transmit an ultrasonic signal to an object in response to a driving signal applied from the transmission and reception modules 113 and 117 and receive an echo signal reflected from the object.

The wireless probe 20 includes the plurality of transducers 115, and the plurality of transducers 115 vibrates depending on transmitted electrical signals and generate ultrasonic waves, which are acoustic energy. As described above, the plurality of transducers 115 may be arranged in two dimensions to form a transducer array.

The transmission module 113 may supply a driving signal to the wireless probe 20 and include a pulse generator 113a, a transmission delayer 113b, and a pulser 113c.

The pulse generator 113a may generate pulses to form transmitted ultrasonic waves depending on a predetermined pulse repetition frequency (PRF), and the transmission delayer 113b may apply a delay time for determining transmission directionality to the pulse. The pulser 113c may apply a driving signal (or driving pulse) corresponding to each of the plurality of transducers 115 with timing corresponding to each pulse to which the delay time is applied.

The reception module 117 may process echo signals received from the plurality of transducers 115 to generate ultrasonic data, and include the amplifier 117a, the main reception circuit 117c, and/or a fifth switch 117b configured to control the connection between the amplifier 117a and the main reception circuit 117c.

The amplifier 117a may amplify an echo signal for each channel and correspond to a low noise amplifier (LNA) that amplifies the echo signal.

The main receiving circuit 117c may include an analog digital converter (ADC), a reception delayer, and/or a summer. The reception delayer may apply a delay time for determining reception directionality to the digitally converted echo signal, and the summer may generate ultrasonic data by summing up the echo signals processed by the reception delayer.

The fifth switch 117b may be provided between the amplifier 117a and the main reception circuit 117c, and the at least one processor may control the connection between the amplifier 117a and the main reception circuit 117c by controlling the fifth switch 117b based on the operation mode of the wireless probe 20.

The auxiliary power supply device 30 may include the auxiliary reception circuit 330 and/or a sixth switch 330a configured to control the connection between the auxiliary reception circuit 330 and the amplifier 117a.

When the wireless probe 20 operates in the predetermined operation mode, the auxiliary reception circuit 330 may process the echo signal received from the transducer 115 to generate ultrasonic data.

The predetermined operation mode may include the CW mode.

For example, when the wireless probe 20 operates in the CW mode, echo signals need to be detected to detect and analyze a blood flow and a movement of a moving object and a frequency of each signal needs to be measured by separating I/Q signals. In addition, a Doppler shift value indicating the frequency shift depending on a movement of an object (e.g., red blood cell) based on the measured frequency needs to be calculated.

Accordingly, the wireless probe 20 may separate the I/Q signals through the auxiliary reception circuit 330 to generate data on frequency shift for measuring a direction and speed of the blood flow, and transmit the data to the image processor 130.

However, the disclosure merely describes a case in which the ultrasonic wireless probe 20 operates in the CW mode as an example to illustrate the auxiliary reception circuit 330, and the auxiliary reception circuit 330 may further include components required when the wireless probe 20 operates in another predetermined mode.

The at least one processor may determine whether the connection of the auxiliary power supply device 30 is activated based on receiving a predetermined operation mode start command, and may turn off the fifth switch 117b and turn on the sixth switch 330a based on the connection of the auxiliary power supply device 30 being activated.

As the fifth switch 117b is turned off and the sixth switch 330a is turned on, the echo signal received through the plurality of transducers 115 may be transmitted to the auxiliary reception circuit 330.

Thereafter, the at least one processor may turn on the fifth switch 117b and turn off the sixth switch 330a based on receiving a predetermined operation mode termination command.

As the fifth switch 117b is turned on and the sixth switch 330a is turned off, the echo signal received through the plurality of transducers 115 may be transmitted to the main reception circuit 117c.

That is, the wireless probe 20 may selectively use the reception circuits to operate in more diverse ultrasonic operation modes, thereby enabling more accurate diagnosis using ultrasonic images.

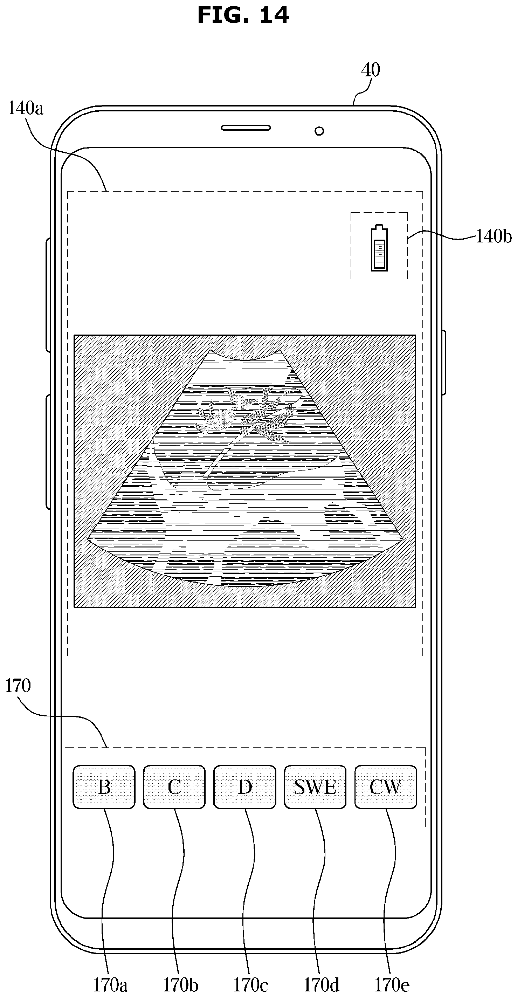

FIGS. 14 to 16 are views illustrating screens displayed on the user display 140 depending on connection activation of the auxiliary power supply device 30, according to an embodiment.

FIG. 14 is a view illustrating a screen displayed on the display 140 according to an embodiment.

Referring to FIG. 14, the display 140 of the ultrasonic diagnostic device 40 wirelessly connected to the wireless probe 20 may display information necessary to operate the ultrasonic diagnostic device 40 in addition to ultrasonic images.

According to an embodiment, the display 140 may include a first display part 140a configured to display an ultrasonic image generated based on data received by the image processor 130 and/or a second display part 140b configured to display the battery capacity of the wireless probe 20.

A position where the second display part 140b is displayed within the display 140 in FIG. 14 is only an example and is not limited thereto, and the second display part 140b may be formed at any position within the display 140.

According to an embodiment, the display 140 may include the input interface 170 configured to receive input from the user.

Referring to FIG. 14, input interfaces 170a to 170e, which are configured to receive a specific mode start input to operate the wireless probe 20 in a specific mode, may be implemented in the form of a touch screen on the display 140.

However, it is only an example that the input interfaces 170a to 170e receiving the specific mode start input are implemented as a touch screen, and any type of input interface may be adopted as long as user input may be received efficiently. For example, the input interfaces 170a to 170e receiving the specific mode start input may be implemented as push buttons.

When the specific mode start input of the user is present in the input interfaces 170a to 170e receiving the specific mode start input, the at least one processor may receive a command for starting a mode corresponding thereto.

Based on this, the at least one processor may control various components of the wireless probe 20 so that the wireless probe 20 may operate in a specific mode.

Accordingly, the at least one processor may control the display 140 to display the generated image on the display part 140a.

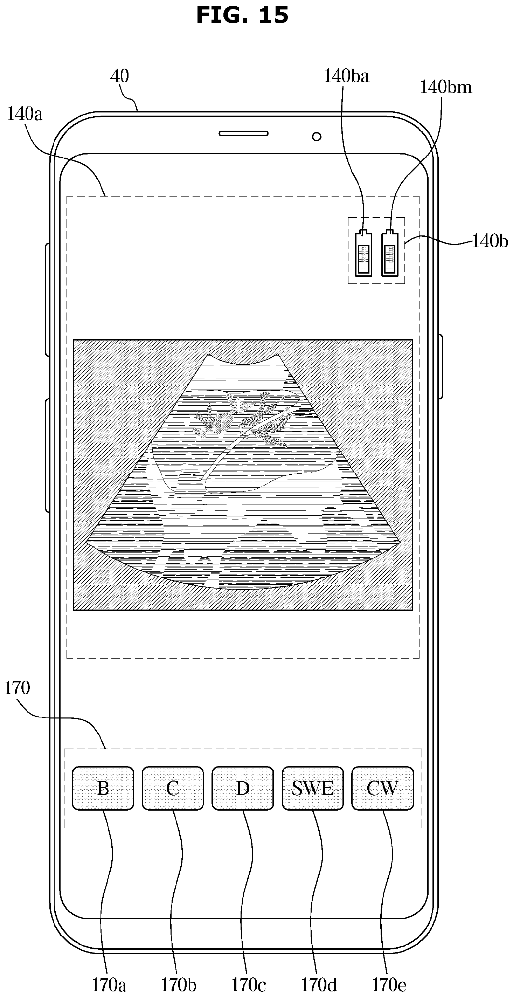

FIG. 15 is a view illustrating a screen displayed on the display 140 when the connection of the auxiliary power supply device 30 is activated according to an embodiment.

According to an embodiment, when the connection of the auxiliary power supply device 30 to the wireless probe 20 is activated, the at least one processor may display a charging capacity of the auxiliary battery 322 on the display 140 separately from the charging capacity of the main battery 114.

For example, referring to FIG. 15, in a state in which the connection of the auxiliary power supply device 30 is deactivated, the at least one processor may display an icon 140ba indicating the charging capacity of the auxiliary battery 322 together with an icon 140bm indicating the charging capacity of the main battery 114 on the second display part 140b displaying the charging capacity of the main battery 114.

The disclosure is not limited to the form of the icon 140ba indicating the charging capacity of the auxiliary battery 322 together with the icon 140bm indicating the charging capacity of the main battery 114, and it is obvious to those skilled in the art that any form is possible as long as it may clearly display information about the charging capacity to the user.

For example, the at least one processor may control the display 140 to display the charging capacity of the main battery 114 and the charging capacity of the auxiliary battery 322 in percentage (%).

The at least one processor may calculate a time during which the wireless probe 20 may operate in the high electric power mode based on the charging capacity of the main battery 114 and the charging capacity of the auxiliary battery 322 and control the display 140 to display an operable time.

The at least one processor not only may display information related to the charging capacity of the main battery 114 on the display 140 and the charging capacity of the auxiliary battery 322, but also may provide information related to the charging capacity of the main battery 114 and/or the charging capacity of the auxiliary battery 322 to the user through audio.

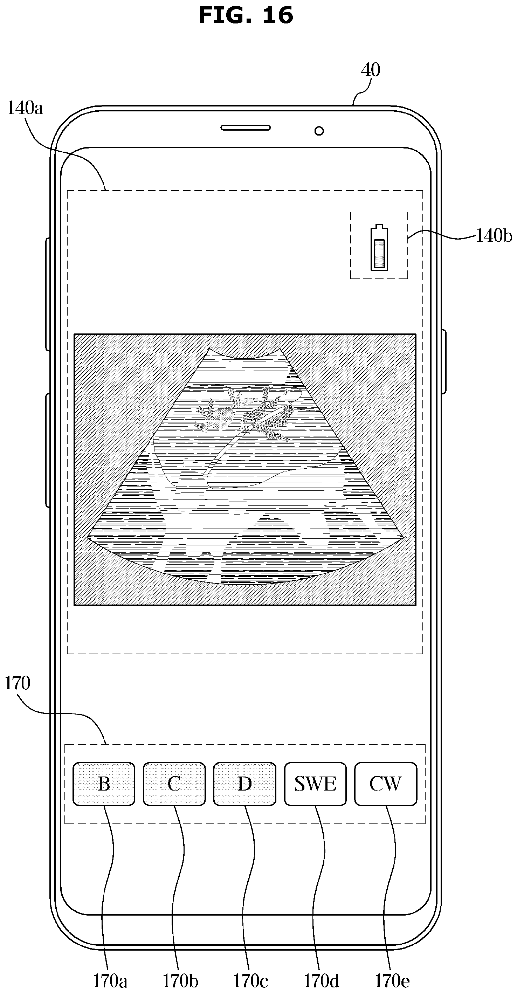

FIG. 16 is a view illustrating a screen displayed on the display 140 when the connection of the auxiliary power supply device 30 is deactivated according to an embodiment.

The at least one processor may deactivate an input interface related to the high electric power mode requiring high electric power based on the connection between the wireless probe 20 and the auxiliary power supply device 30 being deactivated.

In this case, the state in which the connection between the wireless probe 20 and the auxiliary power supply device 30 is deactivated may include a state in which electric power is not supplied from the auxiliary power supply device 30 to the transmission module 113 of the wireless probe 20 even when the wireless probe 20 and the auxiliary electric power supply device 30 are combined as well as physically separated.

When the interface is deactivated, the at least one processor may not receive a specific mode start command even when the user touches the touch screen.

Referring to FIG. 16, the at least one processor may determine whether the connection between the wireless probe 20 and the auxiliary power supply device 30 is deactivated, and deactivate the SWE mode input interface 170d and/or the CW mode interface 170e, which requires high electric power, based on the connection being deactivated.

In addition, the at least one processor may control the display 140 to display a pop-up message indicating that the connection between the wireless probe 20 and the auxiliary power supply device 30 is deactivated, or may transmit information about a connection state of the wireless probe 20 and the auxiliary power supply device 30 to the user through audio.

In the disclosure, various types of information displayed on the display 140 may be transmitted to an external device through a network and displayed on the external device separately or simultaneously with the display 140 of the ultrasonic diagnostic device 40.

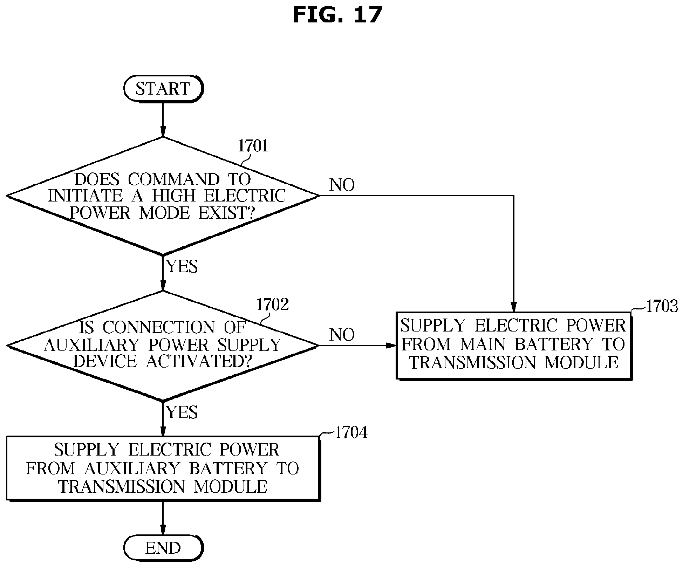

FIG. 17 is an overall control flowchart of the wireless probe 20 and the auxiliary electric power device 30 to supply electric power to the transmission module 113 according to an embodiment.

According to an embodiment, the at least one processor may determine whether the high electric power mode start command exists (1701).

That is, the at least one processor may determine whether a user input for the high electric power mode start command exists.

When the high electric power mode start command exists (YES in 1701), the at least one processor may determine whether the connection of the auxiliary power supply device 30 to the wireless probe 20 is activated (1702).

As described above, that the connection is activated may refer to a state in which the auxiliary power supply device 30 and the wireless probe 20 are combined and electric power may be supplied to the transmission module 113.

When the connection of the auxiliary power supply device 30 is deactivated (NO in 1702), the at least one processor may control the main power source unit 123 to supply electric power from the main battery 114 to the transmission module 113 even when the high electric power mode start command exists (1703).

In addition, when the high electric power mode start command does not exist (NO in 1701) or the connection of the auxiliary power supply device 30 is not activated even if the high electric power mode start command exists (NO in 1702), the at least one processor may control the main power source unit 123 to supply electric power from the main battery 114 to the transmission module 113 (1703).

Specifically, the at least one processor may turn on the first switch 114a of the main power source unit 123 so that electric power is supplied from the main battery 114 to the transmission module 113, and the transmission module 113 may generate a transmission pulse based on the supplied electric power.

Conversely, when the connection of the auxiliary power supply device 30 is activated, the at least one processor may control the auxiliary power source unit 220 to supply electric power from the auxiliary battery 322 to the transmission module 113 (1704).

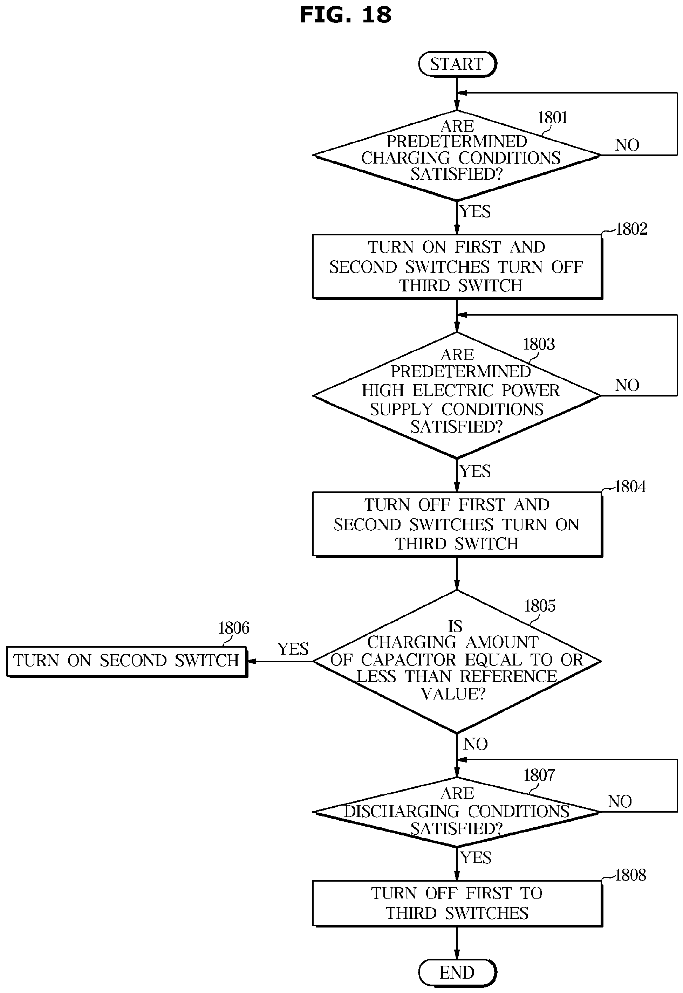

That is, the at least one processor may turn off the first switch 114a to block electric power from being supplied from the main battery 114 to the transmission module 113, and control the second switch 324a and/or the third switch 324b to supply high electric power from the auxiliary battery 322 to the transmission module 113 (1704). A method in which the at least one processor controls the second switch 324a and/or the third switch 324b to supply high electric power from the auxiliary battery 322 to the transmission module 113 will be described in detail below with reference to FIG. 18.

FIG. 18 is a control flowchart for controlling at least one switch to supply electric power to the transmission module 113 according to an embodiment.

According to an embodiment, the at least one processor may determine whether the predetermined charging conditions are satisfied (1801).

In this case, as described above, the predetermined charging conditions may include that the high electric power mode start command is received and that the connection of the power supply device 30 is activated.

In addition, the predetermined charging conditions may include that the high electric power mode start command is received, that the connection of the auxiliary power supply device 30 is activated, and that the charging capacity of the main battery 114 has a value equal to or less than the predetermined value.

When the charging conditions are satisfied (YES in 1801), the at least one processor may turn on the second switch 324a and turn off the third switch 324b to charge the capacitor 326 in the power source circuit 324.

In addition, the at least one processor may turn on the first switch 114a to supply electric power from the main battery 114 to the transmission module 113 while the capacitor 326 is being charged (1802).

After charging of the capacitor 326 begins, the at least one processor may determine whether the high electric power supply conditions are satisfied (1803).

In this case, as described above, the high electric power supply conditions may include that the wireless probe 20 starts operating or is operating in the high electric power mode and that the charging of the capacitor 326 is completed.

In addition, the predetermined high electric power supply conditions may include that the predetermined period of time has passed since the wireless probe 20 operates in the high electric power mode.

When the high electric power supply conditions are satisfied (YES in 1803), the at least one processor may turn off the first switch 114a to block electric power from being supplied from the main battery 114 to the transmission module 113.

In addition, the at least one processor may turn off the second switch 324a to block the connection between the constant current circuit 325 and the capacitor 326, and turn on the third switch 324b to supply the electric energy charged in the capacitor 326 from the capacitor 326 to the transmission module 113 (1804).

Through this, the transmission module 113 may secure the reliability of ultrasonic images by being provided with more stable electric power to generate pulses without distortion.

After turning on the third switch 324b to start supplying the electric energy charged in the capacitor 326 from the capacitor 326 to the transmission module 113, the at least one processor may determine whether a charging amount of the capacitor 326 is equal to or less than a reference value (1805).

In this case, when the charging amount of the capacitor 326 is equal to or less than the reference value (YES in 1805), the at least one processor may turn on the second switch 324a to charge the capacitor 326 from the auxiliary battery 322 through the constant current circuit 325.

As the wireless probe 20 operates in the high electric power mode, because the electric energy charged in the capacitor 326 is sufficient when high electric power is initially supplied to the transmission module 113, a pulse without distortion may be generated in the transmission module 113 with only the electric power supplied from the power source circuit 324 in the high electric power mode.

However, because as time passes, the energy charged in the capacitor 326 decreases and electric power supplied per unit time from the power source circuit 324 to the transmission module 113 also decreases, distortion may be generated in the pulse outputted from the transmission module 113.

Accordingly, as the electrical energy charged in the capacitor 326 decreases, the at least one processor may control the second switch 324a such that electric power is additionally supplied from the constant current circuit 325 to the capacitor 326 by an amount by which the electric power supplied per unit time from the power source circuit 324 to the transmission circuit 113 decreases.

That is, when the electric power supplied per unit time from the capacitor 326 to the transmission module 113 decreases as time passes, the at least one processor may turn on the second switch 324a to supply electric power from the auxiliary battery 322 to the capacitor 326 based on the charging capacity of the capacitor 326. The capacitor 326 may constantly supply high electric power to the transmission module 113 based on the recharged electrical energy.

When the charging amount of the capacitor 326 is not equal to or less than a reference value (YES in 1805), the at least one processor may supply electric power from the capacitor 326 to the transmission module 113 without a charging operation of the capacitor 326 in a state in which the second switch 324a is turned off.

The at least one processor may determine whether discharging conditions are satisfied while electric power is supplied from the capacitor 326 to the transmission module 113 (1807).

In this case, the discharging conditions may include that the wireless probe 20 operates in a mode other than the high electric power mode, or enters an idle state in which the wireless probe 20 does not operate in any mode.

When the discharging conditions are satisfied (YES in 1807), the at least one processor may control the discharge circuit 327 to connect the capacitor 326 to the ground by turning on the fourth switch 324c, thereby discharging the electric energy charged in the capacitor 326.

In addition, the at least one processor may turn off the first switch 114a, the second switch 324a, and the third switch 324b to block charging of the capacitor 326 or block electric power from being supplied to the transmission module 113 while the electric energy charged in the capacitor 326 is discharged (1808).

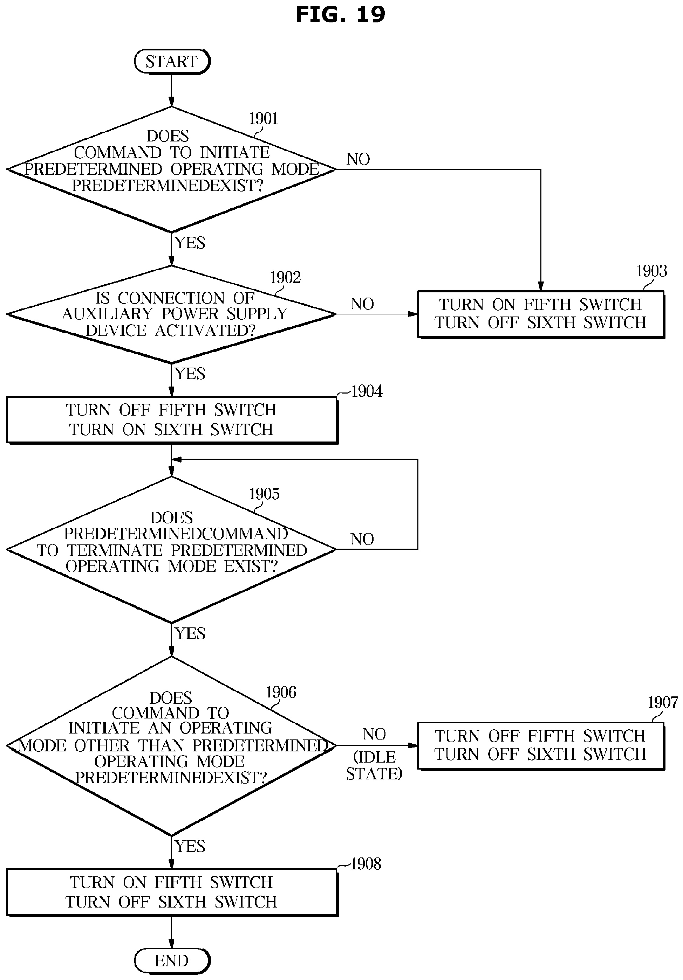

FIG. 19 is a control flowchart for controlling at least one switch to connect the transducer 115 to one of the main reception circuit 117c and the auxiliary reception circuit 330 according to an embodiment.

According to an embodiment, the at least one processor may determine whether the predetermined mode start command exists (1901).

In this case, the predetermined mode may include the CW mode.

When the predetermined mode start command exists (YES in 1801), the at least one processor may determine whether the connection of the auxiliary power supply device 30 is activated (1902).

When the predetermined mode start command does not exist (NO in 1901), or when the connection of the auxiliary power supply device 30 is not activated even if the predetermined mode start command exists (NO in 1902), the at least one processor may turn on the fifth switch 117b and turn off the sixth switch 330a to convert the echo signal received by the transducer 115 into a digital signal through the main reception circuit 117c (1903).

When the predetermined mode start command exists and the connection of the auxiliary power supply device 30 is activated (YES in 1902), the at least one processor may turn off the fifth switch 117b and turn on the sixth switch 330a to convert the echo signal received by the transducer 115 into a digital signal through the auxiliary reception circuit 330 (1904).

The at least one processor may determine whether a predetermined mode termination command exists (1905).

When the predetermined mode termination command exists (YES in 1905), the at least one processor may determine whether a mode start command other than the predetermined mode exists (1906).

In this case, when only the predetermined mode termination command exists and a mode start command other than the predetermined mode does not exist, the wireless probe 20 enters an idle state in which no operation mode is being performed.

In this case, the at least one processor may turn off the fifth switch 117b and the sixth switch 330a (1907).

Conversely, when the predetermined mode termination command exists and a mode start command other than the predetermined mode exists (YES in 1906), the least one processor may turn on the fifth switch 117b and turn off the sixth switch 330a to convert the echo signal received by the transducer 115 into a digital signal through the main reception circuit 330 (1908).

That is, there is an advantage that the wireless probe 20 may have various operation modes by converting the echo signal into a digital signal through an appropriate reception circuit based on the operation mode of the wireless probe 20.

A wireless probe according to an aspect of the disclosure includes a transmission module configured to transmit an ultrasonic signal to an object, a main power source unit configured to supply electric power to the transmission module, an auxiliary power supply device including an auxiliary power source unit configured to supply electric power to the transmission module, and at least one processor configured to control the main power source unit or the auxiliary power source unit to supply electric power to the transmission module from the main power source unit or the auxiliary power source unit in response to a received a command to initiate an operating mode.

The at least one processor may control the main power source unit and the auxiliary power source unit to supply electric power to the transmission module from the auxiliary power source unit based on receiving a high electric power mode start command.

The high electric power mode may include a continuous-wave Doppler mode (hereinafter referred to as CW mode) or a shear wave elastography mode (hereinafter referred to as SWE mode).

The auxiliary power supply device may be connected to or disconnected from the wireless ultrasonic probe by a user.

The main power source unit may include a main battery and a first switch configured to control the connection between the main battery and the transmission module, and the auxiliary power source unit may include an auxiliary battery, an output voltage variable module configured to vary a voltage of the electric power outputted from the auxiliary battery, a capacitor configured to charge electric energy for supplying electric power to the transmission module, a constant current circuit connected to the output voltage variable module to supply a constant current to the capacitor, a second switch configured to control the connection between the constant current circuit and the capacitor, and a third switch configured to control the connection between the capacitor and the transmission module.

A capacity of the auxiliary battery may be equal to or greater than a capacity of the main battery.

The at least one processor may determine whether the connection of the auxiliary power supply device is activated based on receiving the high electric power mode start command,
determine whether predetermined charging conditions are satisfied to start charging the capacitor based on the connection of the auxiliary power supply device being activated, and turn on the first and second switches and turn off the third switch based on the charging conditions being satisfied.

The at least one processor may determine whether predetermined high electric power supply conditions are satisfied to use the electric energy charged in the capacitor after turning on the first and second switches and turning off the third switch, and turn off the first and second switches and turn on the third switch based on the high electric power supply conditions being satisfied.

The auxiliary power source unit may further include a discharge circuit configured to discharge the electric power charged in the capacitor and a fourth switch configured to control the connection between the discharge circuit and the capacitor.

The at least one processor may determine whether predetermined discharging conditions are satisfied to discharge the electric power charged in the capacitor after turning off the first and second switches and turning on the third switch, and turn off the first to third switches and turn on the fourth switch based on the discharging conditions being satisfied.

The wireless probe may further include a reception module including an amplifier configured to amplify the received echo signal, a main reception circuit configured to convert the echo signal amplified by the amplifier into a digital signal, and a fifth switch configured to control the connection between the amplifier and the main reception circuit, wherein the auxiliary power supply device may further include an auxiliary reception circuit configured to convert an echo signal into a digital signal in predetermined operation modes including the CW mode,
and a sixth switch configured to control the connection between the amplifier and the auxiliary reception circuit.

The at least one processor may determine whether the connection of the auxiliary power supply device is activated based on receiving a predetermined operation mode start command, and turn off the fifth switch and turn on the sixth switch based on the connection of the auxiliary power supply device being activated.

The at least one processor may determine whether an operation mode start command other than the predetermined operation mode is received based on receiving a predetermined operation mode termination command, and turn on the fifth switch and turn off the sixth switch based on receiving the operation mode start command other than the predetermined operation mode.

The at least one processor may turn off the fifth and sixth switches based on not receiving an operation mode start command other than the predetermined operation mode.

The at least one processor may transmit a control command to display the capacity of the auxiliary battery based on the connection of the auxiliary power supply device being activated.

The at least one processor may transmit a control command to deactivate an input interface related to the high electric power mode based on the connection of the auxiliary power supply device being deactivated.

An ultrasonic imaging system according to another aspect of the disclosure includes a wireless probe including a transmission module configured to transmit an ultrasonic signal to an object and a main battery configured to supply electric power to the transmission module, an auxiliary power supply device including an auxiliary battery configured to supply electric power to the transmission module and capable of being combined with the wireless probe, and at least one processor configured to control the main battery or the auxiliary battery to supply electric power to the transmission module from the main battery or the auxiliary battery in response to a received operation mode start command, wherein the at least one processor controls the wireless probe and the auxiliary power supply device to supply electric power to the transmission module from the auxiliary battery based on receiving a high electric power mode start command.

The high electric power mode may include a CW mode or a SWE mode.

The ultrasonic imaging system may further include at least one input interface, and at least one display, wherein the at least one processor may control the at least one display to display a capacity of the auxiliary battery based on the connection of the auxiliary power supply device being activated.

The at least one processor may control the at least one input interface to deactivate the at least one input interface related to the high electric power mode based on the connection of the auxiliary power supply device being deactivated.

The disclosed embodiments may be implemented in the form of a recording medium storing instructions executable by a computer. The instructions may be stored in the form of program code, and when executed by a processor, a program module may be created to perform the operations of the disclosed embodiments. The recording medium may be implemented as a computer-readable recording medium.

The computer-readable recording medium includes any type of recording medium in which instructions readable by the computer are stored. For example, the recording medium may include a read only memory (ROM), a random access memory (RAM), a magnetic tape, a magnetic disk, a flash memory, an optical data storage device, and the like.

The embodiments disclosed with reference to the accompanying drawings have been described above. It will be understood by those skilled in the art that various changes in form and details may be made therein without departing from the scope of the disclosure as defined by the appended claims. The disclosed embodiments are illustrative and should not be construed as limiting.

## Claims

1. A wireless probe (20) comprising:
a transmission module (113) configured to transmit an ultrasonic signal to an object;
a main power source unit configured to supply electric power to the transmission module
(113);
an auxiliary power supply device (30) comprising an auxiliary power source unit configured to supply electric power to the transmission module (113); and
at least one processor (118) configured to control the main power source unit and the auxiliary power source unit to supply electric power to the transmission module (113) from the auxiliary power source unit based on receiving a command to initiate a high electric power mode;
wherein the auxiliary power supply device is configured to be connected to or disconnected from the wireless probe by a user,
wherein the main power source unit comprises a main battery (114) and a first switch (114a) configured to control the connection between the main battery (114) and the transmission module (113),
wherein the auxiliary power source unit comprises an auxiliary battery (322), an output voltage variable module (323) configured to vary a voltage of the electric power outputted from the auxiliary battery (322), a capacitor (326) configured to charge electric energy for supplying electric power to the transmission module (113), a constant current circuit (325) connected to the output voltage variable module (323) to supply a constant current to the capacitor (326), a second switch (324a) configured to control the connection between the constant current circuit (325) and the capacitor (326) and a third switch (324b) configured to control the connection between the capacitor (326) and the transmission module (113), and
wherein the at least one processor (118) is further configured to:
determine whether the connection of the auxiliary power supply device (30) is activated based on receiving the command to initiate the high electric power mode,
determine whether predetermined charging conditions for starting charging the capacitor (326) are satisfied based on the connection of the auxiliary power supply device (30) being activated, and
turn on the first and second switches (114a, 324a) and turn off the third switch (324b) based on the charging conditions being satisfied.

2. The wireless probe (20) according to claim 1, wherein
the high electric power mode comprises a Continuous-Wave Doppler mode or a Shear Wave Elastography mode.

3. The wireless probe (20) according to claim 1, wherein
a capacity of the auxiliary battery (322) is equal to or greater than a capacity of the main battery (114).

4. The wireless probe (20) according to claim 1, wherein
the at least one processor (118) configured to:
after turning on the first and second switches (114a, 324a) and turning off the third switch (324b), determine whether predetermined high electric power supply conditions for using the electric energy charged in the capacitor (326) are satisfied, and
turn off the first and second switches (114a, 324a) and turn on the third switch (324b) based on the high electric power supply conditions being satisfied.

5. The wireless probe (20) according to claim 4, wherein
the auxiliary power source unit further comprises:
a discharge circuit (327) configured to discharge the electric power charged in the capacitor (326), and
a fourth switch (324c) configured to control the connection between the discharge circuit (327) and the capacitor (326).

6. The wireless probe (20) according to claim 5, wherein
the at least one processor (118) configured to:
after turning off the first and second switches (114, 324a) and turning on the third switch (324b), determine whether predetermined discharge conditions for discharging the electric power charged in the capacitor (326) are satisfied, and
turn off the first switch (114), the second switch (324a) and the third switch (324b), and turn on the fourth switch (324c) based on the discharge conditions being satisfied.

7. The wireless probe (20) according to claim 2, further comprising:
a reception module (117) comprising:
an amplifier (117a) configured to amplify the received echo signal;
a main reception circuit (117c) configured to convert the echo signal amplified by the amplifier (117a) into a digital signal; and
a fifth switch (117b) configured to control the connection between the amplifier (117a) and the main reception circuit (117c),
wherein the auxiliary power supply device (30) further comprises:
an auxiliary reception circuit (330) configured to convert an echo signal into a digital signal in predetermined operation modes including the continuous-wave Doppler mode; and
a sixth switch (330a) configured to control the connection between the amplifier (117a) and the auxiliary reception circuit (330).

8. The wireless probe (20) according to claim 7, wherein
the at least one processor (118) configured to:
determine whether the connection of the auxiliary power supply device (30) is activated based on receiving a command to initiate an operating mode predetermined, and
turn off the fifth switch (117b) and turn on the sixth switch (330a) based on the connection of the auxiliary power supply device (30) being activated.

9. The wireless probe (20) according to claim 8, wherein
the at least one processor (118) configured to:
determine whether a command to initiate an operating mode other than the predetermined operation mode is received based on receiving a command to terminate an operating mode predetermined, and
turn on the fifth switch (117b) and turn off the sixth switch (330a) based on receiving the command to initiate an operating mode other than the predetermined operation mode.

10. The wireless probe (20) according to claim 9, wherein
the at least one processor (118) configured to turn off the fifth and sixth switches (117b, 330a) based on not receiving the command to initiate an operating mode other than the predetermined operation mode.

11. The wireless probe (20) according to claim 1, wherein
the at least one processor (118) configured to transmit a control command to display the capacity of the auxiliary battery (322) based on the connection of the auxiliary power supply device (30) being activated.

## Patentansprüche

1. Drahtlose Sonde (20) mit:
einem Übertragungsmodul (113), das zum Senden eines Ultraschallsignals an ein Objekt konfiguriert ist;
einer Hauptstromversorgungseinheit, die zum Versorgen des Übertragungsmoduls (113) mit elektrischer Energie konfiguriert ist;
einer Hilfsstromversorgungseinrichtung (30) mit einer Hilfsstromquelleneinheit, die so konfiguriert ist, dass sie das Übertragungsmodul (113) mit Strom versorgt; und
mindestens einen Prozessor (118), der so konfiguriert ist, dass er die Hauptstromquelleneinheit und die Hilfsstromquelleneinheit so steuert, dass das Übertragungsmodul (113) auf der Grundlage eines empfangenen Befehls zum Starten eines Modus mit hoher Stromversorgung von der Hilfsstromquelleneinheit mit Strom versorgt wird;
wobei die Hilfsstromversorgungseinrichtung so konfiguriert ist, dass sie von einem Benutzer mit der drahtlosen Sonde verbunden oder von dieser getrennt werden kann,
wobei die Hauptstromversorgungseinheit eine Hauptbatterie (114) und einen ersten Schalter (114a) umfasst, der so konfiguriert ist, dass er die Verbindung zwischen der Hauptbatterie (114) und dem Übertragungsmodul (113) steuert,
wobei die Hilfsstromversorgungseinheit eine Hilfsbatterie (322) umfasst, ein Ausgangsspannungs-Variationsmodul (323), das so konfiguriert ist, dass es eine Spannung der von der Hilfsbatterie (322) ausgegebenen elektrischen Energie variiert, einen Kondensator (326), der so konfiguriert ist, dass er elektrische Energie zum Versorgen des Übertragungsmoduls (113) mit elektrischer Energie lädt, eine Konstantstromschaltung (325), die mit dem Ausgangsspannungs-Variationsmodul (323) verbunden ist, um den Kondensator (326) mit einem konstanten Strom zu versorgen, einen zweiten Schalter (324a), der so konfiguriert ist, dass er die Verbindung zwischen der Konstantstromschaltung (325) und dem Kondensator (326) steuert, und einen dritten Schalter (324b), der so konfiguriert ist, dass er die Verbindung zwischen dem Kondensator (326) und dem Übertragungsmodul (113) steuert, und
wobei der mindestens eine Prozessor (118) ferner dazu eingerichtet ist, um:
auf der Grundlage des Empfangs des Befehls zum Einleiten des Hochleistungsmodus zu bestimmen, ob die Verbindung der Hilfsstromversorgungseinrichtung (30) aktiviert ist,
auf der Grundlage der aktivierten Verbindung der Hilfsstromversorgungseinrichtung (30) zu bestimmen, ob vorbestimmte Ladebedingungen zum Starten des Ladens des Kondensators (326) erfüllt sind, und
den ersten und zweiten Schalter (114a, 324a) einzuschalten und den dritten Schalter (324b) auszuschalten, basierend auf der Erfüllung der Ladebedingungen.

2. Drahtlose Sonde (20) gemäß Anspruch 1, wobei
der Hochleistungsmodus einen Dauerstrich-Doppler-Modus oder einen Schallwellen-Elastographie-Modus umfasst.

3. Drahtlose Sonde (20) gemäß Anspruch 1, wobei
die Kapazität der Hilfsbatterie (322) gleich oder größer als die Kapazität der Hauptbatterie (114) ist.

4. Drahtlose Sonde (20) gemäß Anspruch 1, wobei
der mindestens eine Prozessor (118) dazu eingerichtet ist, um:
nach dem Einschalten des ersten und zweiten Schalters (114a, 324a) und dem Ausschalten des dritten Schalters (324b) zu bestimmen, ob vorbestimmte Bedingungen für eine hohe Stromversorgung zur Nutzung der im Kondensator (326) gespeicherten elektrischen Energie erfüllt sind, und
auf der Grundlage der Erfüllung der Bedingungen für eine hohe Stromversorgung den ersten und zweiten Schalter (114a, 324a) auszuschalten und den dritten Schalter (324b) einzuschalten.

5. Drahtlose Sonde (20) gemäß Anspruch 4, wobei
die Hilfsstromversorgungseinheit ferner umfasst:
eine Entladungsschaltung (327), die so konfiguriert ist, dass sie die im Kondensator (326) gespeicherte elektrische Energie entlädt, und
einen vierten Schalter (324c), der so konfiguriert ist, dass er die Verbindung zwischen der Entladungsschaltung (327) und dem Kondensator (326) steuert.

6. Drahtlose Sonde (20) gemäß Anspruch 5, wobei
der mindestens eine Prozessor (118) dazu eingerichtet ist, um:
nach dem Ausschalten des ersten und zweiten Schalters (114, 324a) und dem Einschalten des dritten Schalters (324b) zu bestimmen, ob vorbestimmte Entladebedingungen zum Entladen der im Kondensator (326) erfüllt sind, und
den ersten Schalter (114), den zweiten Schalter (324a) und den dritten Schalter (324b) auszuschalten und den vierten Schalter (324c) einzuschalten, wenn die Entladebedingungen erfüllt sind.

7. Drahtlose Sonde (20) gemäß Anspruch 2, die ferner umfasst:
ein Empfangsmodul (117), das umfasst:
einen Verstärker (117a), der so konfiguriert ist, dass er das empfangene Echosignal verstärkt;
eine Hauptempfangsschaltung (117c), die so konfiguriert ist, dass sie das vom Verstärker (117a) verstärkte Echosignal in ein digitales Signal umwandelt; und
einen fünften Schalter (117b), der so konfiguriert ist, dass er die Verbindung zwischen dem Verstärker (117a) und der Hauptempfangsschaltung (117c) steuert,
wobei die Hilfsstromversorgungseinrichtung (30) ferner umfasst:
eine Hilfsempfangsschaltung (330), die so konfiguriert ist, dass sie ein Echosignal in einem vorbestimmten Betriebsmodus, einschließlich des Dauerstrich-Doppler-Modus, in ein digitales Signal umwandelt; und
einen sechsten Schalter (330a), der so konfiguriert ist, dass er die Verbindung zwischen dem Verstärker (117a) und der Hilfsempfangsschaltung (330) steuert.

8. Drahtlose Sonde (20) gemäß Anspruch 7, wobei
der mindestens eine Prozessor (118) dazu eingerichtet ist, um:
auf der Grundlage des Empfangs eines Befehls zum Starten eines vorbestimmten Betriebsmodus zu bestimmen, ob die Verbindung der Hilfsstromversorgungseinrichtung (30) aktiviert ist, und
auf der Grundlage der Aktivierung der Verbindung der Hilfsstromversorgungseinrichtung (30) den fünften Schalter (117b) auszuschalten und den sechsten Schalter (330a) einzuschalten.

9. Drahtlose Sonde (20) nach Anspruch 8, wobei
der mindestens eine Prozessor (118) dazu eingerichtet ist, um:
auf der Grundlage des Empfangs eines Befehls zum Beenden eines vorbestimmten Betriebsmodus zu bestimmen, ob ein Befehl zum Einleiten eines anderen Betriebsmodus als dem vorbestimmten Betriebsmodus empfangen wurde, und
auf der Grundlage des Empfangs des Befehls zum Einleiten eines anderen Betriebsmodus als dem vorbestimmten Betriebsmodus den fünften Schalter (117b) einzuschalten und den sechsten Schalter (330a) auszuschalten.

10. Drahtlose Sonde (20) gemäß Anspruch 9, wobei
der mindestens eine Prozessor (118) so konfiguriert ist, dass er den fünften und den sechsten Schalter (117b, 330a) ausschaltet, basierend darauf, dass er den Befehl zum Starten eines anderen Betriebsmodus als dem vorbestimmten Betriebsmodus nicht empfängt.

11. Drahtlose Sonde (20) gemäß Anspruch 1, wobei
der mindestens eine Prozessor (118) so konfiguriert ist, dass er einen Steuerbefehl zum Anzeigen der Kapazität der Hilfsbatterie (322) auf der Grundlage der aktivierten Verbindung der Hilfsstromversorgungseinrichtung (30) sendet.

## Revendications

1. Sonde sans fil (20) comprenant :
un module de transmission (113) configuré pour transmettre un signal ultrasonique à un objet ;
une unité d'alimentation principale configurée pour fournir de l'énergie électrique au module de transmission (113) ;
un dispositif d'alimentation auxiliaire (30) comprenant une unité d'alimentation auxiliaire configurée pour fournir de l'énergie électrique au module de transmission (113) ; et
au moins un processeur (118) configuré pour commander l'unité d'alimentation principale et l'unité d'alimentation auxiliaire afin de fournir de l'énergie électrique au module de transmission (113) à partir de l'unité d'alimentation auxiliaire sur la base de la réception d'une commande pour lancer un mode à haute puissance électrique ;
dans lequel le dispositif d'alimentation auxiliaire est configuré pour être connecté ou déconnecté de la sonde sans fil par un utilisateur,
dans lequel l'unité d'alimentation principale comprend une batterie principale (114) et un premier commutateur (114a) configuré pour contrôler la connexion entre la batterie principale (114) et le module de transmission (113),
dans lequel l'unité d'alimentation auxiliaire comprend une batterie auxiliaire (322), un module de tension de sortie variable (323) configuré pour faire varier la tension de l'énergie électrique fournie par la batterie auxiliaire (322), un condensateur (326) configuré pour charger de l'énergie électrique afin d'alimenter le module de transmission (113), un circuit à courant constant (325) connecté au module de tension de sortie variable (323) pour fournir un courant constant au condensateur (326), un deuxième commutateur (324a) configuré pour contrôler la connexion entre le circuit à courant constant (325) et le condensateur (326) et un troisième commutateur (324b) configuré pour contrôler la connexion entre le condensateur (326) et le module de transmission (113), et
dans lequel le au moins un processeur (118) est en outre configuré pour :
déterminer si la connexion du dispositif d'alimentation auxiliaire (30) est activée sur la base de la réception de la commande pour lancer le mode haute puissance électrique,
déterminer si les conditions de charge prédéterminées pour démarrer la charge du condensateur (326) sont satisfaites sur la base de l'activation de la connexion du dispositif d'alimentation auxiliaire (30), et
activer les premier et deuxième commutateurs (114a, 324a) et désactiver le troisième commutateur (324b) sur la base du fait que les conditions de charge sont satisfaites.

2. Sonde sans fil (20) selon la revendication 1, dans laquelle
le mode haute puissance électrique comprend un mode Doppler à onde continue ou un mode élastographie à ondes de cisaillement.

3. Sonde sans fil (20) selon la revendication 1, dans laquelle
la capacité de la batterie auxiliaire (322) est égale ou supérieure à la capacité de la batterie principale (114).

4. Sonde sans fil (20) selon la revendication 1, dans laquelle
le au moins un processeur (118) est configuré pour :
après avoir activé les premier et deuxième commutateurs (114a, 324a) et après avoir désactivé le troisième commutateur (324b), déterminer si les conditions prédéterminées d'alimentation électrique élevée pour utiliser l'énergie électrique chargée dans le condensateur (326) sont satisfaites, et
désactiver les premier et deuxième commutateurs (114a, 324a) et activer le troisième commutateur (324b) sur la base du fait que les conditions d'alimentation électrique élevée sont satisfaites.

5. Sonde sans fil (20) selon la revendication 4, dans laquelle
l'unité d'alimentation auxiliaire comprend en outre :
un circuit de décharge (327) configuré pour décharger l'énergie électrique chargée dans le condensateur (326), et
un quatrième commutateur (324c) configuré pour contrôler la connexion entre le circuit de décharge (327) et le condensateur (326).

6. Sonde sans fil (20) selon la revendication 5, dans laquelle
le au moins un processeur (118) est configuré pour :
après avoir désactivé les premier et deuxième commutateurs (114, 324a) et activé le troisième commutateur (324b), déterminer si les conditions de décharge prédéterminées pour décharger l'énergie électrique chargée dans le condensateur (326) sont satisfaites, et
désactiver le premier commutateur (114), le deuxième commutateur (324a) et le troisième commutateur (324b), et activer le quatrième commutateur (324c) sur la base du fait que les conditions de décharge sont satisfaites.

7. Sonde sans fil (20) selon la revendication 2, comprenant en outre :
un module de réception (117) comprenant :
un amplificateur (117a) configuré pour amplifier le signal d'écho reçu ;
un circuit de réception principal (117c) configuré pour convertir le signal d'écho amplifié par l'amplificateur (117a) en un signal numérique ; et
un cinquième commutateur (117b) configuré pour contrôler la connexion entre l'amplificateur (117a) et le circuit de réception principal (117c),
dans lequel le dispositif d'alimentation auxiliaire (30) comprend en outre :
un circuit de réception auxiliaire (330) configuré pour convertir un signal d'écho en un signal numérique dans des modes de fonctionnement prédéterminés, y compris le mode Doppler à onde continue ; et
un sixième commutateur (330a) configuré pour contrôler la connexion entre l'amplificateur (117a) et le circuit de réception auxiliaire (330).

8. Sonde sans fil (20) selon la revendication 7, dans laquelle
le au moins un processeur (118) est configuré pour :
déterminer si la connexion du dispositif d'alimentation auxiliaire (30) est activée sur la base de la réception d'une commande pour lancer un mode de fonctionnement prédéterminé, et
désactiver le cinquième commutateur (117b) et activer le sixième commutateur (330a) sur la base de l'activation de la connexion du dispositif d'alimentation auxiliaire (30).

9. Sonde sans fil (20) selon la revendication 8, dans laquelle
le au moins un processeur (118) est configuré pour :
déterminer si une commande pour lancer un mode de fonctionnement autre que le mode de fonctionnement prédéterminé est reçue sur la base de la réception d'une commande pour terminer un mode de fonctionnement prédéterminé, et
activer le cinquième commutateur (117b) et désactiver le sixième commutateur (330a) sur la base de la réception de la commande pour lancer un mode de fonctionnement autre que le mode de fonctionnement prédéterminé.

10. Sonde sans fil (20) selon la revendication 9, dans laquelle
le au moins un processeur (118) est configuré pour désactiver les cinquième et sixième commutateurs (117b, 330a) sur la base de la non-réception de la commande pour lancer un mode de fonctionnement autre que le mode de fonctionnement prédéterminé.

11. Sonde sans fil (20) selon la revendication 1, dans laquelle
le au moins un processeur (118) est configuré pour transmettre une commande de contrôle afin d'afficher la capacité de la batterie auxiliaire (322) sur la base de l'activation de la connexion du dispositif d'alimentation auxiliaire (30).
